# EUROPÄISCHE PATENTANMELDUNG

(11) **EP 2 070 916 A1**
(43) Veröffentlichungstag der Anmeldung: **17.06.2009**
(21) Anmeldenummer: 07076069.9
(22) Anmeldetag: 10.12.2007
(51) Int. Cl.: C07D 277/32, C07D 417/12, C07D 495/04, C07D 417/14, A61P 37/02, A61K 31/425

(54) **2-Aryl-thiazol-4-carbonsäureamid-Derivate, deren Herstellung und Verwendung als Arzneimittel**

(71) Anmelder: Bayer Schering Pharma Aktiengesellschaft, 13353 Berlin (DE)
(72) Erfinder: Bothe, Ulrich, 10551 Berlin (DE); von Bonin, Arne, 16548 Glienicke-Nordbahn (DE); Nguyen, Duy, 10179 Berlin (DE); Boemer, Ulf, 16548 Glienicke-Nordbahn (DE); Guenther, Gudith, 13187 Berlin (DE)

(57) **Zusammenfassung**

Die vorliegende Erfindung betrifft 2-Arylthiazol-4-carbonsäureamid-Derivate der Formel (I), deren Verwendung als Medikament zur Behandlung verschiedener Erkrankungen, sowie Verfahren zur deren Herstellung

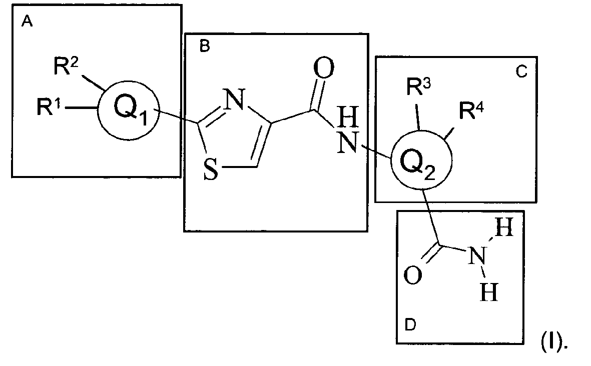

## Beschreibung

Die vorliegende Erfindung betrifft 2-Aryl-thiazol-4-carbonsäureamid-Derivate und deren Verwendung als Medikament zur Behandlung verschiedener Erkrankungen.

### Biologischer Hintergrund

Für zahlreiche chronisch entzündliche Erkrankungen, wie z.B. Rheumatoider Arthritis, Morbus Crohn, Asthma und Multipler Sklerose, ist ein überreagierendes Immunsystem mit verantwortlich. Durch ein vermehrtes Freisetzen von proinflammatorischen Zytokinen kommt es zu Schädigungen von körpereigenen Gewebestrukturen. Hierbei ist das Zusammenspiel von angeborenem und adaptiven Immunsystem von zentraler Bedeutung (Akira et al., 2001). Eine Modulation des Immunsystems mit Substanzen, die mit der Aktivierung von Zellen des angeborenen und/oder des adaptiven Immunsystem interferieren, wirkt anti-inflammatorisch und kann damit den pathologischen Phänotyp in den oben exemplarisch genannten Erkrankungen abmildern.
Die angeborene Immunität ("innate immunity") beruht darauf, daß Mikroorganismen wie Bakterien und Viren bestimmte inherente Merkmale aufweisen, durch die sie vom Immunsystem erkannt werden und dieses anschließend aktivieren. Erkannt werden bestimmte Pathogen assozierte Muster ("pathogen associated molecular pattern, PAMPs"). PAMPs werden von den "Pattern recognition receptors" (PRR) erkannt, zu denen auch Toll Like Rezeptoren (TLR) gehören (Janeway und Medzhitov, 2002). TLRs sind Homologe zum Drosophila-Rezeptorprotein "toll". Der Mensch hat zehn verschiedene TLRs. TLR eins und sechs sind Corezeptoren für TLR2. TLR2 erkennt u.a. Lipoproteine und Lipopeptide. TLR3 erkennt doppelsträngige RNA. TLR4 erkennt u.a. LPS von gram-negativen und Lipoteichonsäure von gram-positiven Bakterien. TLR5 erkennt Flagellin. TLR9 erkennt CpG-Motive in bakterieller DNA (O'Neill, 2006). Corezeptoren können die Erkennungsfähigkeiten von TLRs weiter verändern (Jiang et al., 2005).

### IL-1/-18, TLR Signaltransduktion

TLRs sind in der Signalübertragung mit IL-1 / IL-18 Zytokinrezeptoren verwandt. IL-1 ("endogenes Pyrogen") stimuliert stark Entzündung und induziert Fieber.
Mitglieder der IL-1R/TLR Superfamilie haben eine TIR Domäne (Toll/IL1 Receptor). Die TIR Domäne ist etwa 200 Aminosäuren lang und enthält drei konservierte Sequenzmotive. TIR-Domänen tragende Proteine binden über eine Protein-Protein Interaktion (O'Neill et al., 2005). Die Subklasse eins (IL-1R Familie) enthält drei Igartige-Domänen, der Rezeptor ist ein Heterodimer. Dazu gehören die IL-1 Rezeptoren eins und zwei, der Co-Rezeptor IL-1RAcP und die entsprechenden Proteine des IL-18 Systems. Die Subklasse zwei (TLR-Familie) enthält Leucin-rich Motife. Toll-like Rezeptoren bilden Homo- oder Heterodimere.

Nach Aktivierung der TLR bzw. IL-1, -18 Rezeptoren durch die entsprechenden Liganden wird eine mehrstufige Signalkaskade in Gang gesetzt. Der TLR bzw. IL-1/- 18 Rezeptorkomplex wechselwirkt über TIR/TIR Kontakte mit dem Adaptorprotein MyD88. Die "IL-1 associated receptor kinase" (IRAK-1) hat normalerweise Tollip (Toll interacting protein) gebunden, das wahrscheinlich als ein abschwächendes Molekül ("silencer") wirkt. IRAK/Tollip bindet an den aktiven TLR/IL-1R Komplex. MyD88 verdrängt Tollip wodurch IRAK1 und IRAK-4 aktiviert wird, höchstwahrscheinlich als Dimer durch Transphosphorylierung. Aktives IRAK verlässt den Rezeptor und bindet im Cytoplasma an das Adaptermolekül TRAF (Barton und Medzhitov, 2003). Über TRAF werden weitere Proteine ubiquitiniliert. Über einen unbekannten Mechanismus führt Ub-TRAF zur Autophosphorylierung der S/T-Kinase TAK1 (eine MAP-Kinase Kinasekinase). TAK1 phosphoryliert IκB (NF-κB Aktivierung) und MKK6. Letztere ist für die Aktivierung der MAP-Kinasen p38 und JNK verantwortlich. NF-κB wurde als Nuclear Factor für die Expression der leichten Antikörperkette Kappa in B-Zellen identifiziert, ist aber an der Regulation vieler anderer Gene ebenfalls beteiligt. NF-κB wird im inaktiven Zustand im Cytoplasma zurückgehalten, wo es an den Inhibitor IκB gebunden ist (Deng et al., 2000). Phosphorylierung von IκB bewirkt, daß der Inhibitor IkB proteolytisch abgebaut wird und der Transkriptionsfaktor in den Kern wandern kann. NF-κB ist ein Heterodimer aus den Untereinheiten p65 (Rel) und p50 (Bäuerle und Henkel, 1994). Es gibt mehrere Mitglieder dieser Familie, die auf unterschiedliche Weise zusammenwirken können. NF-κB alleine kann Transkription nicht auslösen. Für Genaktivierung sind transkriptionelle Coaktivatoren erforderlich, wie etwa p300 oder CBT (Akira und Takeda, 2004).
Nach erfolgter Aktivierung von TIR-Domäne enthaltenden Rezeptoren kommt es unter anderem zur Freisetzung von inflammatorischen Zytokinen, wie z.B. IL-1, IL-6, IL-23 und TNF-alpha (Adachi et al., 1998).

Den strukturell naheliegenden Stand der Technik bilden die Strukturen folgender Patentanmeldungen:

In W02007/016292 werden N-Aryl-2-arylthiazol-4-carbonsäureamid-Derivate als Biofilmmodulatoren (Modulatoren von Bakterienfilmen) beschrieben, die aufgrund des bicyclischen C-D-Ringsystemes unterschiedlich von den hier beanspruchten Verbindungen sind.
In W02007/035478 werden Verbindungen offenbart, die anstelle einer Carboxamidgruppe eine Carboxylgruppe in ortho-Positon aufweisen.
In US 6,274,738 werden N-Aryl-2-pyridylthiazol-4-carbonsäureamid-Derivate als Verbindungen beschrieben, die die DNA Primase modulieren. Allerdings weisen diese Verbindungen an der N-Arylgruppe keine Aminocarbonylgruppe auf, die in ortho-Postion bezüglich der Pyridylthiazol-4-carbonsäureamideinheit steht.
Darüber hinaus werden in US 4,879295 N-Tetrazolylthiazolcarboxamide offenbart. Thiazolamidderivate werden in WO2006/122011 als Inhibitoren der Virusreplikation genannt. In W02005/048953 werden Thiazolamidderivate verknüpft mit einer Isoxazoleinheit als Kinaseinhibitoren berschrieben. Die Strukturen unterscheiden sich allerdings von den Strukturen der vorliegenden Erfindung.
Auch die in der W02007/052882 offenbarten Verbindungen weisen keine Aminocarbonylgruppe in ortho-Position zur Aminocarbonylthiazolgruppe auf. Besonderes Merkmal der in W02004072025 beschriebenen Verbindungen ist im Gegensatz zu den hier offenbarten Strukturen ein Pyrrolidinring, der zusätzlich noch mit einem weiteren Substituenten (wie z. B. einer Dimethylaminogruppe) über ein Stickstoffatom verknüpft vorliegt.

In W0200512256, W0200677424, W02006077425 und W0200677428 werden Pyrazolderivate als Kinaseinhibitoren offenbart. Unter anderem sind auch Strukturen beschrieben, bei denen ein Thiazolamid mit der Pyrazoleinheit verknüpft ist, wobei allerdings keines der Pyrazol-Stickstoffatome methyliert vorliegt und die Pyrazoleinheit auch nicht mit einer Aminocarbonylgruppe (CONH₂) substituiert ist.

Pyrazolamide, die aber gleichzeitig mit einer Harnstoffeinheit verknüpft vorliegen, werden in WO2005/37797 beschrieben.

In W02005/115986 werden Pyridinamide beansprucht, wobei aber keine Verknüpfungen mit schwefelhaltigen Heterocyclen wie Thiazol vorgesehen sind.

Strukturell unterschiedlich zu den hier beschriebenen Verbindungen sind auch die in W02005/049604 beschriebenen Pyridinamide, aufgrund der Verknüpfung mit einem sauerstoffsubstituierten Phenylring.

In EP1666455 werden Amide mit zusätzlicher Carboxamidstruktur beschrieben, wobei der Substituent R1 keine Aminocarbonylgruppe sein kann.

Ausgehend von diesem Stand der Technik besteht die Aufgabe der vorliegenden Erfindung, weitere Strukturen für die Therapie bereitzustellen, insbesondere für die Immunmodulation.

Die Aufgabe wird gelöst durch Verbindungen der allgemeinen Formel (I), mit den Bausteinen A, B, C und D , 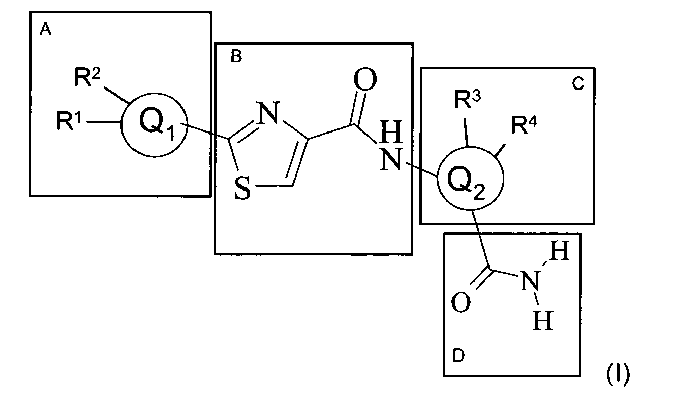 in der
die Bausteine B und D in ortho-Stellung zueinander stehen und
- Q₁: für einen Phenylring oder hydrierten bicyclischen Heteroarylring steht,
- R¹ und R²: unabhängig voneinander stehen für
(i) Wasserstoff, Hydroxy, Nitro, Halogen, Cyano, -CF₃, -NR⁵R⁶ oder
(ii) C(O)-OR¹⁰, -C(O)-R⁷, -C(O)-C₁-C₃-Fluoralkyl, -C(O)-NR⁵R⁶, -NH-C(O)-R⁷ oder
(iii) einen C₁-C₆-Alkyl-, C₂-C₆ -Alkenyl-, C₂-C₆-Alkinyl-, C₁-C₆-Alkoxy-, C₁-C₆-Fluoralkyl- oder einen C₁-C₆-Fluoralkoxyrest,
   jeweils gegebenenfalls mit -C₁-C₃-Alkoxy, Hydroxy, -C(O)-OR¹⁰ oder -NR⁸R⁹ ein- oder mehrfach, gleich oder verschieden substituiert, oder
(iv) O-SO₂-NR⁵R⁶, -SO₂-R⁷, -SO₂-NR⁵R⁶,
- Q₂: für einen Aryl-, Heteroaryl- oder einen hydrierten bizyklischen Heteroarylring steht;
- R³ und R⁴: unabhängig voneinander stehen für
(i) Wasserstoff, Halogen oder -NR¹¹R¹², oder
(ii) einen C₁-C₆-Alkyl- oder C₁-C₆-Alkoxyrest, die jeweils gegebenenfalls ein- oder mehrfach, gleich oder verschieden mit Hydroxy, C₁-C₃-Alkoxy, -NR⁸R⁹ oder
Heterocyclyl substituiert sind, wobei der Heterocylylring ein- oder mehrfach, gleich oder verschieden substituiert sein kann mit C₁-C₃-Alkyl oder -CO-R⁷, wobei
- R⁵ und R⁶: unabhängig voneinander stehen für Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder-CO-R⁷, oder
- R⁵ und R⁶: bilden alternativ gemeinsam mit dem Stickstoffatom einen 5- bis 7-gliedrigen Ring, der gegebenenfalls zusätzlich zum Stickstoffatom ein weiteres Heteroatom enthält und der gegebenenfalls mit C₁-C₆-Alkyl und/oder mit -C(O)-R⁷ ein- oder mehrfach, gleich oder verschieden substituiert ist, und
- R⁷: für einen C₁-C₆ Alkylrest steht, und
- R⁸, R⁹, R¹⁰: unabhängig voneinander stehen für Wasserstoff oder einen C₁-C₆ Alkylrest, und
- R¹¹ und R¹²: unabhängig voneinander stehen für Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder-CO-R⁷, oder
- R¹¹ und R¹²: bilden alternativ gemeinsam mit dem Stickstoffatom einen 5- bis 7-gliedrigen Ring, der gegebenenfalls zusätzlich zum Stickstoffatom ein weiteres Heteroatom enthält und der gegebenenfalls mit C₁-C₆-Alkyl und/oder mit -C(O)-R⁷ ein- oder mehrfach, gleich oder verschieden substituiert ist,
sowie deren Salze, Enantiomere und Diastereomere.

Der Erfindung liegen folgende Definitionen zu Grunde:

### Cₙ-Alkyl:

Monovalenter, geradkettiger oder verzweigter, gesättigter Kohlenwasserstoffrest mit n Kohlenstoffatomen.

### Ein C₁-C₆ Alkylrest umfasst unter anderem beispielsweise:

Methyl-, Ethyl-, Propyl-, Butyl-, Pentyl-,Hexyl-, *iso*-Propyl-, *iso*-Butyl-, sec-Butyl, *tert-*Butyl-, iso-Pentyl-, 2-Methylbutyl-, 1-Methylbutyl-, 1-Ethylpropyl-, 1,2-Dimethylpropyl, *neo-*Pentyl-, 1,1-Dimethylpropyl-, 4-Methylpentyl-, 3-Methylpentyl-, 2-Methylpentyl-, 1-Methylpentyl-, 2-Ethylbutyl-, 1-Ethylbutyl-, 3,3-Dimethylbutyl-, 2,2-Dimethylbutyl-, 1,1-Dimethylbutyl-, 2,3-Dimethylbutyl-, 1,3-Dimethylbutyl- 1,2-Dimethylbutyl-. Bevorzugt ist ein Methyl-, Ethyl-, Propyl- oder Isopropylrest.

### Cₙ-Fluoralkyl:

Monovalenter, geradkettiger oder verzweigter, gesättigter, vollständig oder teilweise fluorierter Kohlenwasserstoffrest mit n Kohlenstoffatomen.

### Ein C₁-C₆ Fluoralkylrest umfasst unter anderem beispielsweise:

Trifluormethyl, Difluormethyl, Monofluormethyl, Pentafluorethyl, Heptafluorpropyl, 2,2,2-Trifluorethyl, 3,3,3-Trifluorpropyl, 5,5,6,6,6-Pentafluorhexyl, Pentafluorallyl, 1,1,1,3,3,3-Hexafluor-2-propyl.

Bevorzugt ist ein Trifluormethyl, 2,2,2-Trifluorethyl und Pentafluorethylrest.

### Cₙ-Alkenyl:

monovalenter, geradkettiger oder verzweigter Kohlenwasserstoffrest mit n Kohlenstoffatomen und mindestens einer Doppelbindung.

### Ein C₂-C₆ Alkenylrest umfasst unter anderem beispielsweise:

Vinyl-, Allyl-, (E)-2-Methylvinyl-, (Z)-2-Methylvinyl-, Homoallyl-, (E)-But-2-enyl-, (Z)-But-2-enyl-, (E)-But-1-enyl-, (Z)-But-1-enyl-, Pent-4-enyl-, (E)-Pent-3-enyl-, (Z)-Pent-3-enyl-, (E)-Pent-2-enyl-, (Z)-Pent-2-enyl-, (E)-Pent-1-enyl-, (Z)-Pent-1-enyl-, Hex-5-enyl-, (E)-Hex-4-enyl-, (Z)-Hex-4-enyl-, (E)-Hex-3-enyl-, (Z)-Hex-3-enyl-, (E)-Hex-2-enyl-, (Z)-Hex-2-enyl-, (E)-Hex-1-enyl-, (Z)-Hex-1-enyl-, Isopropenyl-, 2-Methylprop-2-enyl-, 1-Methylprop-2-enyl-, 2-Methylprop-1-enyl-, (E)-1-Methylprop-1-enyl-, (Z)-1-Methylprop-1-enyl-, 3-Methylbut-3-enyl-, 2-Methylbut-3-enyl-, 1-Methylbut-3-enyl-, 3-Methylbut-2-enyl-, (E)-2-Methylbut-2-enyl-, (Z)-2-Methylbut-2-enyl-, (E)-1-Methylbut-2-enyl-, (Z)-1-Methylbut-2-enyl-, (E)-3-Methylbut-1-enyl-, (Z)-3-Methylbut-1-enyl-, (E)-2-Methylbut-1-enyl-,
(Z)-2-Methylbut-1-enyl-, (E)-1-Methylbut-1-enyl-, (Z)-1-Methylbut-1-enyl-, 1,1-Dimethylprop-2-enyl-, 1-Ethylprop-1-enyl-, 1-Propylvinyl-, 1-Isopropylvinyl-, 4-Methylpent-4-enyl-, 3-Methylpent-4-enyl-, 2-Methylpent-4-enyl-, 1-Methylpent-4-enyl-, 4-Methylpent-3-enyl-, (E)-3-Methylpent-3-enyl-, (Z)-3-Methylpent-
3-enyl-, (E)-2-Methylpent-3-enyl-, (Z)-2-Methylpent-3-enyl-, (E)-1-Methylpent-3-enyl-, (Z)-1-Methylpent-3-enyl-, (E)-4-Methylpent-2-enyl-, (Z)-4-Methylpent-2-enyl-, (E)-3-Methylpent-2-enyl-, (Z)-3-Methylpent-2-enyl-, (E)-2-Methylpent-2-enyl-, (Z)-2-Methylpent-2-enyl-, (E)-1-Methylpent-2-enyl-, (Z)-1-Methylpent-2-enyl-, (E)-4-Methylpent-1-enyl-, (Z)-4-Methylpent-1-enyl-, (E)-3-Methylpent-1-enyl-, (Z)-3-Methylpent-1-enyl-, (E)-2-Methylpent-1-enyl-, (Z)-2-Methylpent-1-enyl-, (E)-1-Methylpent-1-enyl-, (Z)-1-Methylpent-1-enyl-, 3-Ethylbut-3-enyl-, 2-Ethylbut-3-enyl-, 1-Ethylbut-3-enyl-, (E)-3-Ethylbut-2-enyl-, (Z)-3-Ethylbut-2-enyl-, (E)-2-Ethylbut-2-enyl-, (Z)-2-Ethylbut-2-enyl-, (E)-1-Ethylbut-2-enyl-, (Z)-1-Ethylbut-2-enyl-, (E)-3-Ethylbut-1-enyl-, (Z)-3-Ethylbut-1-enyl-, 2-Ethylbut-1-enyl-, (E)-1-Ethylbut-1-enyl-, (Z)-1-Ethylbut-1-enyl-, 2-Propylprop-2-enyl-, 1-Propylprop-2-enyl-, 2-Isopropylprop-2-enyl-, 1-Isopropylprop-2-enyl-, (E)-2-Propylprop-1-enyl-, (Z)-2-Propylprop-1-enyl-, (E)-1-Propylprop-1-enyl-, (Z)-1-Propylprop-1-enyl-, (E)-2-Isopropylprop-1-enyl-, (Z)-2-Isopropylprop-1-enyl-, (E)-1-Isopropylprop-1-enyl-, (Z)-1-Isopropylprop-1-enyl-, (E)-3,3-Dimethylprop-1-enyl-, (Z)-3,3-Dimethylprop-1-enyl-, 1-(1,1-Dimethylethyl)ethenyl.

Bevorzugt ist ein Vinyl- oder Allylrest.

### Cₙ-Alklnyl:

Monovalenter, geradkettiger oder verzweigter Kohlenwasserstoffrest mit n Kohlenstoffatomen und mindestens einer Dreifachbindung.

### Ein C₂-C₆ Alkinylrest umfasst unter anderem beispielsweise:

Ethinyl-, Prop-1-inyl-, Prop-2-inyl-, But-1-inyl-, But-2-inyl-, But-3-inyl-, Pent-1-inyl-, Pent-2-inyl-, Pent-3-inyl-, Pent-4-inyl-, Hex-1-inyl-, Hex-2-inyl-, Hex-3-inyl-, Hex-4-inyl-, Hex-5-inyl-, 1-Methylprop-2-inyl-, 2-Methylbut-3-inyl-, 1-Methylbut-3-inyl-, 1-Methylbut-2-inyl-, 3-Methylbut-1-inyl-, 1-Ethylprop-2-inyl-, 3-Methylpent-4-inyl, 2-Methylpent-4-inyl, 1-Methylpent-4-inyl, 2-Methylpent-3-inyl, 1-Methylpent-3-inyl, 4-Methylpent-2-inyl, 1-Methylpent-2-inyl, 4-Methylpent-1-inyl, 3-Methylpent-1-inyl, 2-Ethylbut-3-inyl-, 1-Ethylbut-3-inyl-, 1-Ethylbut-2-inyl-, 1-Propylprop-2-inyl-, 1-Isopropylprop-2-inyl-, 2,2-Dimethylbut-3-inyl-, 1,1-Dimethylbut-3-inyl-, 1,1-Dimethylbut-2-inyl- oder eine 3,3-Dimethylbut-1-inyl-.
Bevorzugt ist ein Ethinyl-, Prop-1-inyl- oder Prop-2-inylrest.

### Cₙ-Cycloalkyl:

Monovalenter, cyclischer Kohlenwasserstoffring mit n Kohlenstoffatomen.

### C₃-C₇-Cyclolalkylring umfasst:

Cyclopropyl, Cyclobutyl, Cyclopentyl, Cyclohexyl- und Cycloheptyl.

Bevorzugt ist ein Cyclopropyl-, Cyclobutyl-, Cylopentyl- oder ein Cyclohexylring.

### Cₙ-Alkoxy:

Geradkettiger oder verzweigter Cₙ-Alkyletherrest der Formel -OR mit R=Alkyl.

### Cₙ-Fluoralkoxy:

Geradkettiger oder verzweigter Cₙ-Fluoralkyletherrest der Formel -OR mit R=Cₙ-Fluoralkyl.

### Cₙ-Aryl

Cₙ-Aryl ist ein monovalentes, aromatisches Ringsystem ohne Heteroatom mit n Kohlenwasserstoffatomen.
C₆-Aryl ist gleich Phenyl. C₁₀-Aryl ist gleich Naphthyl.
Bevorzugt ist Phenyl.

### Heteroatome

Unter Heteroatomen sind Sauerstoff-, Stickstoff- oder Schwefel-Atome zu verstehen.

### Heteroaryl

Heteroaryl ist ein monovalentes, aromatisches Ringsystem mit mindestens einem von einem Kohlenstoff verschiedenen Heteroatom. Als Heteroatome können Stickstoffatome, Sauerstoffatome und/oder Schwefelatome vorkommen. Die Bindungsvalenz kann an einem beliebigen aromatischen Kohlenstoffatom oder an einem Stickstoffatom sein.

Ein monocyclischer Heteroarylring gemäß der vorliegenden Erfindung hat 5 oder 6 Ringatome.

Heteroarylringe mit 5 Ringatomen umfassen beispielsweise die Ringe: Thienyl, Thiazolyl, Furanyl, Pyrrolyl, Oxazolyl, Imidazolyl, Pyrazolyl, Isoxazolyl, Isothiazolyl, Oxadiazolyl, Triazolyl, Tetrazolyl und Thiadiazolyl.

### Heteroarylringe mit 6 Ringatomen umfassen beispielsweise die Ringe:

Pyridinyl, Pyridazinyl, Pyrimidinyl, Pyrazinyl und Triazinyl.

Ein bicyclischer Heteroarylring gemäß der vorliegenden Erfindung hat 9 bis 10 Ringatome.

### Heteroarylringe mit 9 Ringatomen umfassen beispielsweise die Ringe:

Indolyl, Isoindolyl, Indazolyl, Benzothiazolyl, Benzofuranyl, Benzothiophenyl, Benzimidazolyl, Benzoxazolyl, Azocinyl, Indolizinyl, Purinyl.

### Heteroarylringe mit 10 Ringatomen umfassen beispielsweise die Ringe:

Isochinolinyl, Chinolinyl, Cinnolinyl, Chinazolinyl, Chinoxalinyl, Phthalazinyl, 1,7- oder 1,8-Naphthyridinyl, Pteridinyl

Monocyclische Heteroarylringe mit 5 oder 6 Ringatomen sind bevorzugt.

Hydrierte bicyclische Aryl- oder Heteroarylringe sind Bizyklen, bei denen ein Ring teilweise oder vollständig hydriert vorliegt. Die Bindungsvalenz kann an einem beliebigen Atom des aromatischen Teiles des hydrierten bicyclischen Aryl- oder Heteroarylringes sein.

### Hydrierte bicyclische Aryl- oder Heteroarylringe umfassen beispielsweise die Ringe:

Indanyl, 1,2,3,4-Tetrahydro-naphthalenyl, 1,2,3,4-Tetrahydro-isochinolinyl, 1,2,3,4-Tetrahydro-chinolinyl, 2,3-Dihydro-1H-indolyl, 2,3-Dihydro-1H-isoindolyl, 4,5,6,7-Tetrahydro-thieno[2,3-c]pyridinyl, 4,5,6,7-Tetrahydro-thieno[3,2-c]pyridinyl, 2,3-Dihydro-benzofuranyl, Benzo[1,3]dioxolyl, 2,3-Dihydro-benzo[1,4]dioxinyl, Chromanyl, 1,2,3,4-Tetrahydro-chinoxalinyl, 3,4-Dihydro-2H-benzo[1,4]oxazinyl.

Hydrierte bicyclische Aryl- oder Heteroarylringe bei denen ein Ring teilweise oder vollständig hydriert vorliegt, können gegebenenfalls eine oder zwei Carbonylgruppen im Ringsystem enthalten.

Beispiele hierfür sind Indolonyl, Isoindolonyl, Benzoxazinonyl, Phthalazinonyl, Chinolonyl, Isochinolonyl, Phthalidyl, Thiophthalidyl.

### Heterocyclyl

Heterocyclyl im Sinne der Erfindung ist ein vollständig hydriertes Heteroaryl (vollständig hydriertes Heteroaryl = gesättigtes Heterocyclyl) d.h. ein nicht aromatisches Ringsystem mit mindestens einem von einem Kohlenstoff verschiedenen Heteroatom. Als Heteroatome können Stickstoffatome, Sauerstoffatome und/oder Schwefelatome vorkommen. Die Bindungsvalenz kann an einem beliebigen Kohlenstoffatom oder an einem Stickstoffatom sein.

### Heterocycylring mit 3 Ringatomen umfasst beispielsweise:

Aziridinyl.

### Heterocycylring mit 4 Ringatomen umfasst beispielsweise:

Azetidinyl, Oxetanyl.

### Heterocycylringe mit 5 Ringatomen umfassen beispielsweise die Ringe:

Pyrrolidinyl, Imidazolidinyl , Pyrazolidinyl und Tetrahydrofuranyl.

### Heterocyclylringe mit 6 Ringatomen umfassen beispielsweise die Ringe:

Piperidinyl, Piperazinyl, Morpholinyl, Tetrahydropyranyl und Thiomorpholinyl

### Heterocycylringe mit 7 Ringatomen umfassen beispielsweise:

Azepanyl, Oxepanyl, [1,3]-Diazepanyl, [1,4]-Diazepanyl.

### Heterocycylringe mit 8 Ringatomen umfassen beispielsweise:

Oxocanyl, Azocanyl.

Heterocyclylringe können gegebenenfalls teilweise ungesättigt sein und/oder auch eine Carbonylgruppe im Ring enthalten.
Beispiele hierfür sind Dihydro-furan-2-onyl, Pyrrolidin-2-onyl, Piperazin-2-onyl, Morpholin-2-onyl, 3(2H)-Pyridazinonyl, 5,6-Dihydro-2-pyran-2-onyl, 5,6-Dihydropyridin-2(1H)-onyl, 2-Piperidonyl, 1,2,3,6-Tetrahydropyridinyl.

### Halogen

Die Bezeichnung Halogen umfasst Fluor, Chlor, Brom und Iod.

Ebenfalls als von der vorliegenden Erfindung als erfasst anzusehen sind alle Verbindungen, die sich ergeben durch jede mögliche Kombination der oben genannten möglichen, bevorzugten und besonders bevorzugten Bedeutungen der Substituenten.

Besondere Ausführungsformen der Erfindung bestehen darüber hinaus in Verbindungen, die sich durch Kombination der direkt in den Beispielen offenbarten Bedeutungen für die Substituenten ergeben.

Ebenfalls als von der vorliegenden Erfindung als erfasst anzusehen sind die Salze der Verbindungen.

Die Formulierung der erfindungsgemäßen Verbindungen zu pharmazeutischen Präparaten erfolgt in an sich bekannter Weise, indem man den oder die Wirkstoffe mit den in der Galenik gebräuchlichen Hilfsstoffen in die gewünschte Applikationsform überführt.

Als Hilfsstoffe können dabei beispielsweise Trägersubstanzen, Füllstoffe, Sprengmittel, Bindemittel, Feuchthaltemittel, Gleitmittel, Ab- und Adsorptionsmittel, Verdünnungsmittel, Lösungsmittel, Cosolventien, Emulgatoren, Lösungsvermittler, Geschmackskorrigentien, Färbemittel, Konservierungs-, Stabilisierungs-, Netzmittel, Salze zur Veränderung des osmotischen Drucks oder Puffer zum Einsatz kommen. Dabei ist auf Remington's Pharmaceutical Science, 15th ed. Mack Publishing Company, East Pennsylvania (1980) hinzuweisen.

Die pharmazeutischen Formulierungen können
in fester Form, zum Beispiel als Tabletten, Dragees, Pillen, Suppositorien, Kapseln, transdermale Systeme oder
in halbfester Form , zum Beispiel als Salben, Cremes, Gele, Suppositiorien, Emulsionen oder
in flüssiger Form, zum Beispiel als Lösungen, Tinkturen, Suspensionen oder Emulsionen vorliegen.

Hilfsstoffe im Sinne der Erfindung können beispielsweise Salze, Saccharide (Mono-, Di-, Tri-, Oligo-, und/oder Polysaccharide), Proteine, Aminosäuren, Peptide, Fette, Wachse, Öle, Kohlenwasserstoffe sowie deren Derivate sein, wobei die Hilfsstoffe natürlichen Ursprungs sein können oder synthetisch bzw. partial synthetisch gewonnen werden können.

Für die orale oder perorale Applikation kommen insbesondere Tabletten, Dragees, Kapseln, Pillen, Pulver, Granulate, Pastillen, Suspensionen, Emulsionen oder Lösungen in Frage.
Für die parenterale Applikation kommen insbesondere Suspensionen, Emulsionen und vor allem Lösungen in Frage.

Aufgrund ihrer anti-entzündlichen und zusätzlichen immunsuppressiven Wirkung können die erfindungsgemäßen Verbindungen der allgemeinen Formel (I) als Medikamente zur Behandlung oder Prophylaxe folgender Krankheitszustände bei Säugetieren und Menschen, für die lokale und systemische Applikation Verwendung finden:
(i) Lungenerkrankungen, die mit entzündlichen, allergischen und / oder proliferativen Prozessen einhergehen:
   - Chronisch obstruktive Lungenerkrankungen jeglicher Genese, vor allem Asthma bronchiale
   - Bronchitis unterschiedlicher Genese
   - Adult respiratory distress syndrome (ARDS), akutes Atemnotssyndrom
   - Bronchiektasen
   - Alle Formen der restriktiven Lungenerkrankungen, vor allem allergische Alveolitis,
   - Lungenödem, insbesondere allergisches
   - Sarkoidosen und Granulomatosen, insbesondere Morbus Boeck
(ii) Rheumatische Erkrankungen / Autoimmunerkrankungen / Gelenkerkrankungen, die mit entzündlichen, allergischen und / oder proliferativen Prozessen einhergehen:
   - Alle Formen rheumatischer Erkrankungen, insbesondere rheumatoide Arthritis, akutes rheumatisches Fieber, Polymyalgia rheumatica, Morbus Behcet
   - Reaktive Arthritis
   - Entzündliche Weichteilerkrankungen sonstiger Genese
   - Arthritische Symtome bei degenerativen Gelenkerkrankungen (Arthrosen)
   - Vitiligo
   - Kollagenosen jeglicher Genese, z.B. systemischer Lupus erythematodes, Sklerodermie, Polymyositis, Dermatomyositis- Sjögren-Syndrom, Still-Syndrom, Felty-Syndrom
   - Sarkoidosen und Granulomatosen
   - Weichteilrheumatismus
(iii) Allergien oder pseudoallergische Erkrankungen, die mit entzündlichen, und / oder proliferativen Prozessen einhergehen:
   - alle Formen allergischer Reaktionen, z.B. Quincke Ödem, Heuschnupfen, Insektenstich, allergische Reaktionen auf Arzneimittel, Blutderivate, Kontrastmittel etc., Anaphylaktischer Schock, Urtikaria, allergische und irritative Kontakdermatitis, allergische Gefäßerkrankungen
   - Vasculitis allergica
(iv) Gefäßentzündungen (Vaskulitiden)
   - Panarteriitis nodosa, Arteriitis temporalis, Erythema nodosum
   - Polyarteritis nodosa
   - Wegner Granulomatose
   - Riesenzellarteriitis
(v) Dermatologische Erkrankungen, die mit entzündlichen, allergischen und / oder proliferativen Prozessen einhergehen:
   - Atopische Dermatitis (vor allem bei Kindern)
   - Sämtliche Ekzemformen wie z.B. atopisches Ekzem (v.a. bei Kindern)
   - Exantheme jedweder Genese oder Dermatosen
   - Psoriasis und Parapsoriasis-Formenkreis
   - Pityriasis rubra pilaris
   - Erythematöse Erkrankungen, ausgelöst durch unterschiedlichen Noxen, z.B. Strahlen, Chemikalien, Verbrennungen etc.
   - Bullöse Dermatosen wie z.B. autoimmuner Pemphigus vulgaris, bullöses Pemphigoid
   - Erkrankungen des lichenoiden Formenkreises,
   - Pruritus (z. B. allergischer Genese)
   - Rosacea-Formenkreis
   - Erythema exsudativum multiforme
   - Manifestation von Gefäßerkrankungen
   - Haarausfall wie Alopecia areata
   - Kutane Lymphome
(vi) Nierenerkrankungen, die mit entzündlichen, allergischen und / oder proliferativen Prozessen einhergehen:
   - Nephrotisches Syndrom
   - Alle Nephritiden, z.B. Glomerulonephritis
(vii) Lebererkrankungen, die mit entzündlichen, allergischen und / oder proliferativen Prozessen einhergehen:
   - akute Hepatitis unterschiedlicher Genese
   - chronisch aggressive und / oder chronisch intermittierende Hepatitis
(viii) Gastrointestinale Erkrankungen, die mit entzündlichen, allergischen und / oder proliferativen Prozessen einhergehen:
   - regionale Enteritis (Morbus Crohn)
   - Colitis Ulcerosa
   - Gastroenteritiden anderer Genese, z.B. einheimische Sprue
(ix) Augenerkrankungen, die mit entzündlichen, allergischen und / oder proliferativen Prozessen einhergehen:
   - allergische Keratitis, Uveitis, Iritis,
   - Konjunktivitis
   - Blepharitis
   - Neuritis nervi optici
   - Chorioiditis
   - Ophthalmia sympathica
(x) Erkrankungen des Hals-Nasen-Ohren-Bereiches, die mit entzündlichen, allergischen und / oder proliferativen Prozessen einhergehen:
   - allergische Rhinitis, Heuschnupfen
   - Otitis externa, z.B. bedingt durch Kontaktekzem
(xi) Neurologische Erkrankungen, die mit entzündlichen, allergischen und / oder proliferativen Prozessen einhergehen:
   - Hirnödem, vor allem allergisches Hirnödem
   - Multiple Sklerose
   - akute Encephalomyelitis
   - Meningitis, vor allem allergische
   - Guillain-Barre Syndrom
   - Morbus Alzheimer
(xii) Bluterkrankungen, die mit entzündlichen, allergischen und / oder proliferativen Prozessen einhergehen wie z. B.: M. Hodgkin oder Non-Hodgkin Lymphome, Thrombozytaemien, Erythrozytosen
   - Erworbene hämolytische Anämie
   - Idiopathische Thrombozytopenie
   - Idiopathische Granulozytopenie
(xiii) Tumorerkrankungen, die mit entzündlichen, allergischen und / oder proliferativen Prozessen einhergehen
   - Akute lymphatische Leukämie
   - Maligne Lymphome
   - Lymphogranulomatosen
   - Lymphosarkome
(xiv) Endokrine Erkrankungen, die mit entzündlichen, allergischen und / oder proliferativen Prozessen einhergehen wie z. B.:
   - Endokrine Orbitopathie
   - Thyreoiditis de Quervain
   - Hashimoto Thyreoiditis
   - Morbus Basedow
   - Granulomatous thyroiditis
   - Struma lymphomatosa
   - Autoimmune adrenalitis
   - Diabetes mellitus, insbesondere Typ 1 Diabetes
(xv) Organ- und Gewebstransplantationen, Graft-versus-host-disease
(xvi) Schwere Schockzustände, z.B anaphylaktischer Schock, systemic inflammatory response syndrome (SIRS)

Ein Gegenstand der Erfindung ist die Verwendung der erfindungsgemäßen Verbindungen der allgemeinen Formel (I) zur Herstellung eines Arzneimittels.

Ein weiterer Gegenstand der Erfindung ist die Verwendung der erfindungsgemäßen Verbindungen zur Behandlung von Erkrankungen, die mit entzündlichen, allergischen und / oder proliferativen Prozessen einhergehen.

In der allgemeinen Formel (I) kann Q₁ für einen Phenylring oder hydrierten bicyclischen Heteroarylring stehen.

Bevorzugt steht Q₁ für einen für einen Phenyl-, Benzodioxolyl-, 3,4-Methylendioxyphenyl-, 2,3-Dihydrobenzofuranyl-, 3,4-Dihydro-2H-benzo[1,4]oxazin-7-yl-, Indanonyl- oder einen 2,3-Dihydroindolylring.

Besonders bevorzugt steht Q₁ für einen Phenyl-, 3,4-Methylendioxyphenyl-, 2,3-Dihydrobenzofuranyl- oder einen 3,4-Dihydro-2H-benzo[1,4]oxazin-7-ylring.

In der allgemeinen Formel (I) können R¹ und R² unabhängig voneinander stehen für:
(i) Wasserstoff, Hydroxy, Nitro, Halogen, Cyano, -CF₃, -NR⁵R⁶ oder
(ii) C(O)-OR¹⁰, -C(O)-R⁷, -C(O)-C₁-C₃-Fluoralkyl, -C(O)-NR⁵R⁶, -NH-C(O)-R⁷ oder
(iii) einen C₁-C₆-Alkyl-, C₂-C₆ -Alkenyl-, C₂-C₆-Alkinyl-, C₁-C₆-Alkoxy-, C₁-C₆-Fluoralkyl- oder einen C₁-C₆-Fluoralkoxyrest,
   jeweils gegebenenfalls mit -C₁-C₃-Alkoxy, Hydroxy, -C(O)-OR¹⁰ oder -NR⁸R⁹ ein- oder mehrfach, gleich oder verschieden substituiert, oder
(iv) O-SO₂-NR⁵R⁶, -SO₂-R⁷, -SO₂-NR⁵R⁶.

Bevorzugt stehen R¹ und R² unabhängig voneinander für:
(i) Wasserstoff, Hydroxy, Halogen, Cyano, -CF₃, -NR⁵R⁶ oder
(ii) einen C₁-C₆-Alkyl-, C₁-C₆-Alkoxy-, C₁-C₆-Fluoralkyl- oder einen C₁-C₆-Fluoralkoxyrest oder
(iii) SO₂-R⁷,

Besonders bevorzugt stehen R¹ und R² unabhängig voneinander für:
(i) Wasserstoff, Halogen, oder
(ii) einen C₁-C₆-Alkoxy-, C₁-C₆-Alkylrest, oder
(iii) SO₂-R⁷.

In der allgemeinen Formel (I) kann Q₂ stehen für einen Aryl-, Heteroaryl- oder einen hydrierten bizyklischen Heteroarylring.

### Bevorzugt steht Q₂ für einen:

Phenyl-, Thienyl-, Thiazolyl-, Furanyl-, Pyrrolyl-, Oxazolyl-, Imidazolyl-, Pyrazolyl-, Isoxazolyl, Isothiazolyl, 1,2,3-Triazolyl, Pyridinyl-, Pyridazinyl-, Pyrimidinyl-, Pyrazinyl-, 1,2,3,4-Tetrahydro-isochinolinyl-, 1,2,3,4-Tetrahydro-chinolinyl-, 2,3-Dihydro-1H-indolyl-, 2,3-Dihydro-1H-isoindolyl-, 4,5,6,7-Tetrahydro-thieno[2,3-c]pyridinyl-, 4,5,6,7-Tetrahydro-thieno[3,2-c]pyridinyl-, 2,3-Dihydro-benzofuranyl-, Benzo[1,3]dioxolyl-, 2,3-Dihydro-benzo[1,4]dioxinyl-, 1,2,3,4-Tetrahydro-chinoxalinyl- oder einen 3,4-Dihydro-2H-benzo[1,4]oxazinylring.

Besonders bevorzugt steht Q₂ für einen Phenyl-, Pyrazolyl- oder einen 4,5,6,7-Tetrahydro-thieno[2,3-c]pyridinylring.

In der allgemeinen Formel (I) können R³ und R⁴ unabhängig voneinander stehen für:
(i) Wasserstoff, Halogen oder -NR¹¹R¹², oder
(ii) einen C₁-C₆-Alkyl- oder C₁-C₆-Alkoxyrest, die jeweils gegebenenfalls ein- oder mehrfach, gleich oder verschieden mit Hydroxy, C₁-C₃-Alkoxy, -NR⁸R⁹ oder Heterocyclyl substituiert sind, wobei der Heterocylylring ein- oder mehrfach, gleich oder verschieden substituiert sein kann mit C₁-C₃-Alkyl oder -CO-R⁷,

Bevorzugt stehen R³ und R⁴ unabhängig voneinander für:
(i) Wasserstoff, Halogen, -NR¹¹R¹² oder
(ii) einen C₁-C₆-Alkyl- oder C₁-C₆-Alkoxyrest, jeweils gegebenenfalls mit Morpholin oder -NR⁸R⁹ substituiert.

Besonders bevorzugt stehen R³ und R⁴ unabhängig voneinander für Wasserstoff, Halogen, C₁-C₆-Alkyl oder -NR¹¹R¹².

In der allgemeinen Formel (I) können R⁵ und R⁶ unabhängig voneinander stehen für: für Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder -CO-R⁷, oder
- R⁵ und R⁶: bilden alternativ gemeinsam mit dem Stickstoffatom einen 5- bis 7- gliedrigen Ring, der gegebenenfalls zusätzlich zum Stickstoffatom ein weiteres Heteroatom enthält und der gegebenenfalls mit C₁-C₆-Alkyl und/oder mit -C(O)-R⁷ ein- oder mehrfach, gleich oder verschieden substituiert ist.

Bevorzugt stehen R⁵ und R⁶ unabhängig voneinander für Wasserstoff oder einen C₁-C₆ Alkylrest, der gegebenenfalls ein- oder mehrfach, gleich oder verschieden mit Hydroxy, -NR⁸R⁹, Halogen oder C₁-C₃-Alkoxy substituiert sein kann.

Besonders bevorzugt stehen R⁵ und R⁶ unabhängig voneinander für Wasserstoff oder einen C₁-C₄-Alkylrest.

In der allgemeinen Formel (I) kann R⁷ stehen für einen C₁-C₆ Alkylrest.

Bevorzugt steht R⁷ für einen C₁-C₄-Alkylrest.

Besonders bevorzugt steht R⁷ für einen C₁-C₃-Alkylrest.

In der allgemeinen Formel (I) können R⁸, R⁹, R¹⁰ unabhängig voneinander stehen für Wasserstoff oder einen C₁-C₆-Alkylrest.

Bevorzugt stehen R⁸ und R⁹ unabhängig voneinander für Wasserstoff oder einen C₁-C₄-Alkylrest und R¹⁰ für Wasserstoff.

Besonders bevorzugt stehen R⁸ und R⁹ unabhängig voneinander für Wasserstoff oder einen C₁-C₃-Alkylrest und R¹⁰ für Wasserstoff

In der allgemeinen Formel (I) können R¹¹und R¹² unabhängig voneinander stehen für: Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder -CO-R⁷,
oder
R¹¹ und R¹² bilden alternativ gemeinsam mit dem Stickstoffatom einen 5- bis 7-gliedrigen Ring, der gegebenenfalls zusätzlich zum Stickstoffatom ein weiteres Heteroatom enthält und der gegebenenfalls mit C₁-C₆-Alkyl und/oder mit -C(O)-R⁷ ein- oder mehrfach, gleich oder verschieden substituiert ist.

Bevorzugt stehen R¹¹ und R¹² unabhängig voneinander für Wasserstoff oder einen C₁-C₆ Alkylrest, der gegebenenfalls ein- oder mehrfach, gleich oder verschieden mit Morpholin oder durch -N(CH₃)₂, -NH-CH₃ oder -NH-C₂H₅ substituiert sein kann, oder
R¹¹ und R¹² bilden alternativ gemeinsam mit dem Stickstoffatom einen 5- bis7-gliedrigen Ring, der gegebenenfalls zusätzlich zum Stickstoffatom ein weiteres Heteroatom enthält und der gegebenenfalls mit C₁-C₄-Alkyl und/oder mit -C(O)-R⁷ ein- oder mehrfach, gleich oder verschieden substituiert ist.

Besonders bevorzugt bilden R¹¹ und R¹² gemeinsam mit dem Stickstoffatom einen 6-gliedrigen Ring, der gegebenenfalls zusätzlich zum Stickstoffatom ein weiteres Heteroatom enthält und der mit C₁-C₄-Alkyl ein- oder mehrfach, gleich oder verschieden substituiert sein kann.

Eine bevorzugte Untergruppe bilden Verbindungen der Formel (I) mit den Bausteinen A, B, C und D,
in der die Bausteine B und D in ortho-Stellung zueinander stehen und
- Q₁: für einen Phenyl-, Benzodioxolyl-, 3,4-Methylendioxyphenyl-, 2,3-Dihydrobenzofuranyl-, 3,4-Dihydro-2H-benzo[1,4]oxazin-7-yl-, Indanonyl- oder einen 2,3-Dihydroindolylring steht,
- R¹ und R²: unabhängig voneinander stehen für
(i) Wasserstoff, Hydroxy, Halogen, Cyano, -CF₃, -NR⁵R⁶ oder
(ii) einen C₁-C₆-Alkyl-, C₁-C₆-Alkoxy-, C₁-C₆-Fluoralkyl- oder einen C₁-C₆-Fluoralkoxyrest oder
(iii) SO₂-R⁷,
- Q₂: für einen Phenyl-, Thienyl-, Thiazolyl-, Furanyl-, Pyrrolyl-, Oxazolyl-, Imidazolyl-, Pyrazolyl-, Isoxazolyl, Isothiazolyl, 1,2,3-Triazolyl, Pyridinyl-, Pyridazinyl-, Pyrimidinyl-, Pyrazinyl-, 1,2,3,4-Tetrahydro-isochinolinyl-, 1,2,3,4-Tetrahydro-chinolinyl-, 2,3-Dihydro-1H-indolyl-, 2,3-Dihydro-1H-isoindolyl-, 4,5,6,7-Tetrahydro-thieno[2,3-c]pyridinyl-, 4,5,6,7-Tetrahydro-thieno[3,2-c]pyridinyl-, 2,3-Dihydro-benzofuranyl-, Benzo[1,3]dioxolyl-, 2,3-Dihydro-benzo[1,4]dioxinyl-, 1,2,3,4-Tetrahydro-chinoxalinyl- oder einen 3,4-Dihydro-2H-benzo[1,4]oxazinylring steht,
- R³ und R⁴: unabhängig voneinander stehen für
(i) Wasserstoff, Halogen, -NR¹¹R¹² oder
(ii) einen C₁-C₆-Alkyl- oder C₁-C₆-Alkoxyrest,
jeweils gegebenenfalls mit Morpholin oder -NR⁸R⁹ substituiert,
wobei
- R⁵ und R⁶: unabhängig voneinander stehen für Wasserstoff oder einen C₁-C₆ Alkylrest, der gegebenenfalls ein- oder mehrfach, gleich oder verschieden mit Hydroxy, -NR⁸R⁹, Halogen oder C₁-C₃-Alkoxy substituiert sein kann, und
- R⁷: für einen C₁-C₄ Alkylrest steht, und
- R⁸ und R⁹: unabhängig voneinander stehen für Wasserstoff oder einen C₁-C₄ Alkylrest,
- R¹¹ und R¹²: unabhängig voneinander stehen für Wasserstoff oder einen C₁-C₆ Alkylrest, der gegebenenfalls ein- oder mehrfach, gleich oder verschieden mit Morpholin oder durch -N(CH₃)₂, -NH-CH₃ oder -NH-C₂H₅ substituiert sein kann, oder
- R¹¹ und R¹²: bilden alternativ gemeinsam mit dem Stickstoffatom einen 5- bis 7-gliedrigen Ring, der gegebenenfalls zusätzlich zum Stickstoffatom ein weiteres Heteroatom enthält und der gegebenenfalls mit C₁-C₄-Alkyl und/oder mit -C(O)-R⁷ ein- oder mehrfach, gleich oder verschieden substituiert ist,
sowie deren Salze, Enantiomere und Diastereomere.

Besonders bevorzugt sind Verbindungen der allgemeinen Formel (I) mit den Bausteinen A, B, C und D
in der
die Bausteine B und D in ortho-Stellung zueinander stehen und
- Q₁: für einen für Phenyl-, 3,4-Methylendioxyphenyl-, 2,3-Dihydrobenzofuranyl- oder einen 3,4-Dihydro-2H-benzo[1,4]oxazin-7-ylring steht,
- R¹ und R²: unabhängig voneinander stehen für
(i) Wasserstoff, Halogen, oder
(ii) einen C₁-C₆-Alkoxy-, C₁-C₆-Alkylrest, oder
(iii) SO₂-R⁷
- Q₂: für einen für einen Phenyl-, Pyrazolyl- oder einen 4,5,6,7-Tetrahydrothieno[2,3-c]pyridinylring steht,
- R³ und R⁴: unabhängig voneinander stehen für Wasserstoff, Halogen, C₁-C₆-Alkyl oder -NR¹¹R¹²,
wobei
- R⁷: für einen C₁-C₃-Alkylrest steht, und
- R¹¹ und R¹²: gemeinsam mit dem Stickstoffatom einen 6-gliedrigen Ring bilden, der gegebenenfalls zusätzlich zum Stickstoffatom ein weiteres Heteroatom enthält und der mit C₁-C₄-Alkyl ein- oder mehrfach, gleich oder verschieden substituiert sein kann,
sowie deren Salze, Enantiomere und Diastereomere.

### Herstellung der erfindungsgemäßen Verbindungen

### Verfahrensvariante 1:

### Herstellung der erfindungsgemäßen Verbindungen durch Amidkupplung Endproduktes

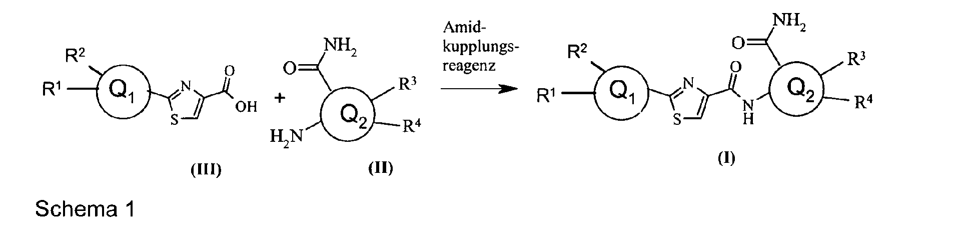

Die Darstellung der erfindungsgemäßen Verbindungen der allgemeinen Formel (I) kann durch Umsetzung der Intermediate gemäß Formel (III) mit Verbindungen der Fomel II erfolgen durch ein Amidkupplungsreagenz (siehe Publikation Chemfiles, Peptide Synthesis, 2007, 7, Nr. 2 der Firma Sigma-Aldrich). Hierbei können zum Beispiel Carbodiimide (zum Beispiel N-Cyclohexyl-N'-(2-morpholinoethyl)carbodiimid metho-p-toluolsulfonat CAS2491-17-0) oder Uroniumsalze (zum Beispiel 0-(7-Azabenzotriazol-1-yl)-N,N,N'N'-tetramethyluroniumhexafluorophosphat (HATU)) verwendet werden in Kombination mit einer Base wie zum Beispiel Pyridin. Hierbei haben Q₁, Q₂, R¹, R², R³ und R⁴ die in der allgemeinen Formel (I) gemäß der Ansprüche 1 bis 7 angegebenen Bedeutungen.

### Verfahrensvariante 2:

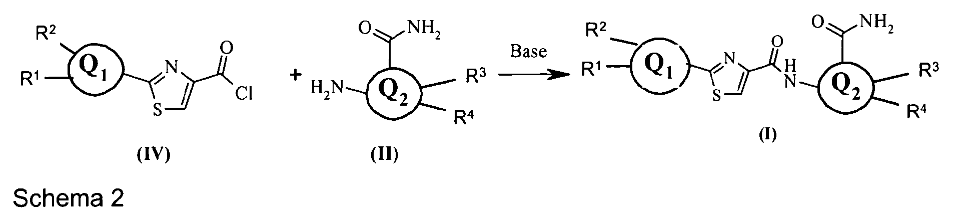

Die Darstellung der erfindungsgemäßen Verbindungen der allgemeinen Formel (I) erfolgt bei dieser Variante durch die Umsetzung der Intermediate gemäß Formel (IV) mit Verbindungen der Fomel (II) in Gegenwart einer Base wie zum Beispiel Triethylamin. Hierbei haben Q₁, Q₂, R¹, R², R³ und R⁴ die in der allgemeinen Formel (I) gemäß der Ansprüche 1 bis 7 angegebenen Bedeutungen.

### Verfahrensvariante 3:

### a) Herstellung von Intermediaten der Formel (V)

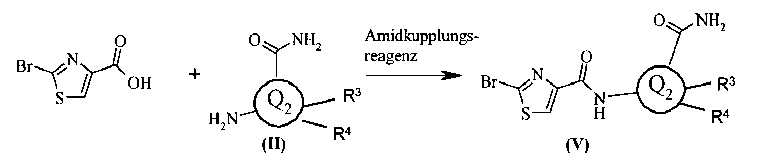

2-Bromthiazol-4-carbonsäure wird mit Intermediaten der Formel (II) durch ein Amidkupplungsreagenzien wie bei Verfahrensvariante 1 beschrieben zu Intermediaten der Formel (V) umgesetzt.

### b) Herstellung des Endproduktes

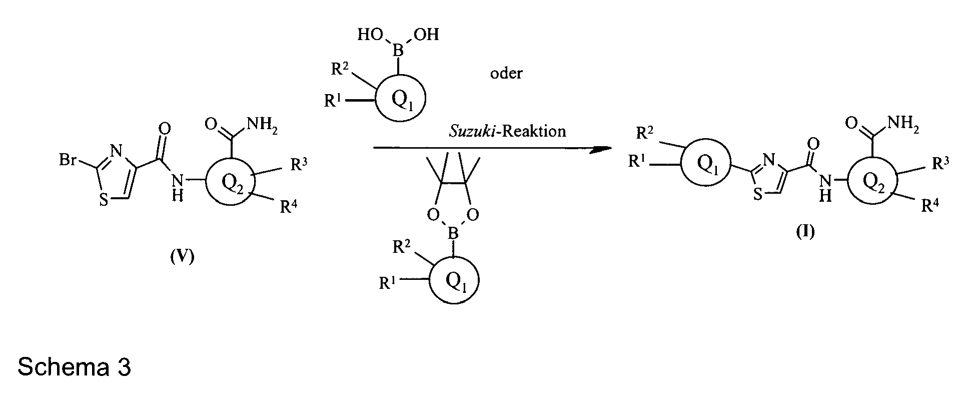

Intermediate der Formel (V) werden mit Boronsäuren oder Boronsäurepinakolestern im Rahmen einer *Suzuki-Miyaura-Reaktion* zu den erfindungsgemäßen Verbindungen der Formel (I) umgesetzt. Als Katalysatoren bzw. Liganden kommen dabei zum Beispiel die in N. Miyaura, A. Suzuki, Chem. Rev.; 1995; 95(7); 2457-2483 oder in C. J. O'Brien et. al. Chem. Eur. J. 2006, 12, 4743 - 4748 sowie die in der Publikation Chemfifes, Catalysis, 2007, 7, Nr. 5 von der Firma Sigma-Aldrich beschrieben Katalysatoren bzw. Katalysatorensysteme in Betracht. Hierbei haben Q₁, Q₂, R¹, R², R³ und R⁴ die in der allgemeinen Formel (I) gemäß der Ansprüche 1 bis 7 angegebenen Bedeutungen.

### Reinigung der erfindungsgemäßen Verbindungen

In einigen Fällen können die erfindungsgemäßen Verbindungen durch präparative HPLC zum Beispiel durch ein Autopurifier-Gerät der Firma Waters (Detektion der Verbindungen durch UV-Detektion sowie Elektrospray-Ionisation) in Kombination mit kommerziell erhältlichen, vorgepackten HPLC Säulen (zum Beispiel Säule XBridge (Firma Waters), C18, 5µm, 30 x 100mm) gereinigt werden. Als Lösemittelsystem kann Acetonitril / Wasser +0,1 % Trifluoressigsäure verwendet werden (Fluss 50 ml / min). Zum Entfernen des HPLC-Lösemittelgemisches kann eine Gefriertrocknung oder eine Zentrifugation erfolgen. Die so erhaltenen Verbindungen können als Trifluoressigsäure (TFA)-Salze vorliegen und können zu den jeweiligen freien Basen durch dem Fachmann bekannte Standard-Laborprozeduren überführt werden. In einigen Fällen können die erfindungsgemäßen Verbindungen durch Chromatographie an Kieselgel gereinigt werden. Hierbei können zum Beispiel vorgepackte Silica Gel Cartridges (zum Beispiel von der Firma Separtis, *Isolute*® *Flash silica* ge/) in Kombination mit dem Flashmaster II Chromatograhiegerät (Argonaut/Biotage) und Chromatographielösemittel bzw. -Gemische wie zum Beispiel Hexan, Essigester sowie Dichlormethan und Methanol in Betracht.

### Strukturanalytik der erfindungsgemäßen Verbindungen:

In einigen Fällen werden die erfindungsgemäßen Verbindungen durch LC-MS analysiert: Retentionszeiten Rₜ aus der LC-MS-Analytik: Detektion: UV = 200 - 400 nm *(Acquity* HPLC-System der Firma *Waters*)/ MS 100-800 Daltons; 20 V (Micromass / *Waters* ZQ 4000) im ESI⁺-Modus (zur Erzeugung positiv geladener Molekülionen); HPLC-Säule: X Bridge *(Waters),* 2.1 x 50 mm, BEH 1.7 µm; Flussmittel: A: H2O/0.05% HCOOH, B: CH3CN/0.05% HCOOH. Gradient: 10-90% B in 1.7 min, 90% B für 0.2 min, 98-2% B in 0.6 min; Flussrate: 1.3 ml / min. Die Angabe *LC-MS (ZQ)* bezieht sich auf die Verwendung eines *Waters* ZQ4000 Gerätes und die Angabe *LC-MS (SQD)* auf die Benutzung eines Single Quadrupol API (Atomic Pressure lonization) Massendetektors *(Waters)* zur Aufnahme eines Massenspektrums.

### Verfahrensvariante 1

### Beispiel 1.1

### N-[2-(Aminocarbonyl)-4-(4-methyl-piperazin-1-yl)phenyl]-2-(3-chlorphenyl)-4-thiazolcarboxamid

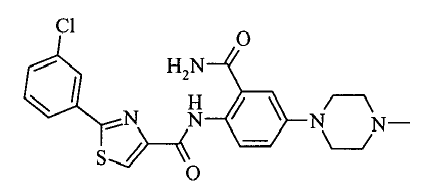

### Stufe a: Herstellung 5-(4-Methyl-piperazin-1-yl)-2-nitro -benzamid

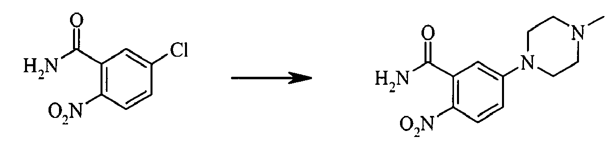

5-Chlornitrobenzamid (7 g) wird in THF vorgelegt und N-Methylpiperazin (4.3 ml, 1.1 Äquivalente) per Spritze zugegeben und unter Rückfluß 22 Stunden erhitzt. Dann werden erneut 1.1 Äq. N-Methylpiperazin zugegeben und 26 h unter Rückfluß erhitzt. Danach gibt man Tetrabutylammoniumfluorid (5 ml, 1 M in THF) und 1.0 Äq. N-Methylpiperazin zu und erhitzt erneut 27.5 h. Man kühlt auf Raumtemperatur ab und saugt den ausgefallenen Feststoff ab. Nach Waschen mit Tetrahydrofuran und Trocknen im Vakuum erhält man 5-(4-Methyl-piperazin-1-yl)-2-nitro -benzamid als Feststoff (9.4 g, Rohprodukt).

1 H-NMR (400 MHz, D6-DMSO, ausgewählte Signale): δ = 6.82 (d, 1 H), 6.97 (dd, 1 H), 7.48 (br. s, 1 H), 7.83 (br. s, 1 H), 7.90 (d, 1 H).

### Stufe b: Herstellung 2-Amino-5-(4-methyl-piperazin-1-yl)-benzamid

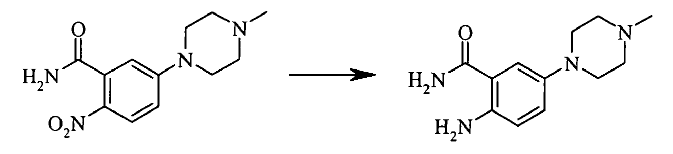

Das Rohprodukt aus Stufe a wird mit Ethanol (188 ml) Palladium auf Kohle (10%ig, wasserfeucht) versetzt. Danach wird bei Raumtemperatur in einer Wasserstoffatmosphäre 1.5 h gerührt. Der Katalysator wird abfiltriert und das Ethanol am Rotationsverdampfer entfernt. Man erhält 2-Amino-5-(4-methyl-piperazin-1-yl) -benzamid als Feststoff (Rohprodukt).

1 H-NMR (400 MHz, D6-DMSO): δ = 2.78 (s, 3H), 2.9 - 3.7 (m, verdeckt durch Wassersignal), 6.0 - 6.5 (br s), 6.65 (d, 1 H), 6.95 (dd, 1 H), 7.07 (br. s, 1 H), 7.11 (d, 1 H), 7.81 (br. s, 1 H)

### Stufe c: Herstellung des Endproduktes

Man legt 2-(3-Chlorphenyl)thiazol-4-carbonsäure (240 mg) und das Produkt aus Stufe b (234 mg) in DMF (5 ml) und Triethylamin (0.28 ml) vor, addiert HATU (494 mg) und rührt bei Raumtemperatur 20 h. Danach gießt man auf Wasser (20 ml), rührt um und saugt den entstandenen Feststoff ab und wäscht den Feststoff mit Essigester und Diethylether. Der Feststoff wird dann in einer Mischung aus Toluol und Aceton aufgelöst und das Aceton am Rotationsverdampfer bis zum Ausfallen eines Feststoffes entfernt. Der Feststoff wurde abgesaugt und mit Essigester und Ether gewaschen. Nach Reinigung durch präparative HPLC erhielt man N-[2-Aminocarbonyl-4-(4-methyl-piperazin-1-yl)phenyl]-2-(3-chlorpheny)-4-thiazolcarboxamid als weissen Feststoff.

### Bedingungen für die präparative HPLC:

Säule: XBridge (Firma Waters) C18 5µ 150x30 mm,

Lösemittel A: H2O / 0.1 % HCOOH, Lösemittel B: Acetonitril,

Gradient: 0 min 99%A 1 %B, 1.00 min 99%A 1 %B, 7.50 min 1 %A 99%B, 10.00 min 1%A 99%B, Flussrate: 50.0 mL/min, Detektion: DAD (200-400 nm) TAC ; MS-ESI+ (m/z=160-1000 m/z) TIC

Temperatur: Rt

1 H-NMR (300 MHz, D6-DMSO, Formiatsalz): 2.28 (s, 3H), 2.54 (m, verdeckt durch Lösemittelsignal), 3.21 (m, 4H), 7.18 (m, 1 H), 7.34 (m, 1 H), 7.61 (m, 2H), 7.75 (br. s., 1 H), 8.06 (m, 1 H), 8.16 (m, 2H), 8.30 (br. s., 1 H), 8.49 (s, 1 H), 8.58 (d, 1 H), 12.9 (s, 1 H)

### Beispiel 1.2

### N-[2-Aminocarbonyl-4-(4-methyl-piperazin-1-yl)-phenyl]-2-phenyl-4-thiazolcarboxamid

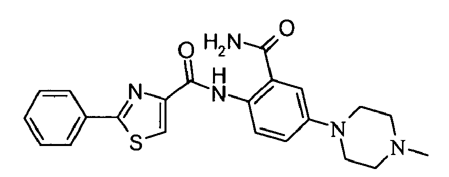

Analog zu Beispiel 1.1 erhält man N-[2-Aminocarbonyl-4-(4-methyl-piperazin-1-yl)-phenyl]-2-phenyl-4-thiazolcarboxamid durch die Reaktion von 2-Phenylthiazol-4-carbonsäure (205 mg) und 2-Amino-5-(4-methyl-piperazin-1-yl)-benzamid (234 mg) und nachfolgende Reinigung durch präparative HPLC (Bedingungen analog Beispiel 1.1).
1 H-NMR (300 MHz, D6-DMSO, Formiatsalz): 2.28 (s, 3H), 2.54 (m, verdeckt durch Lösemittelsignal), 3.21 (m, 4H), 7.18 (m, 1 H), 7.34 (m, 1 H), 7.57 (m, 3H), 7.81 (br. s., 1H), 8.11 (m, 2H), 8.17 (s, 1 H), 8.30 (br. s., 1 H), 8.44 (s, 1 H), 8.61 (d, 1 H), 12.9 (s, 1 H)

### Beispiel 1.3

### 2-(3-Methansulfonyl-phenyl)-thiazol-4-carbonsäure (2-carbamoyl-4 -(morpholin-4-yl)-phenyl)-amid

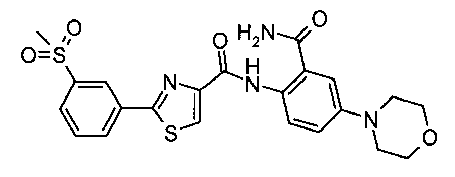

### Stufe a: Herstellung 2-(3-Methansulfonyl-phenyl)-thiazol-4-carbonsäureethylester

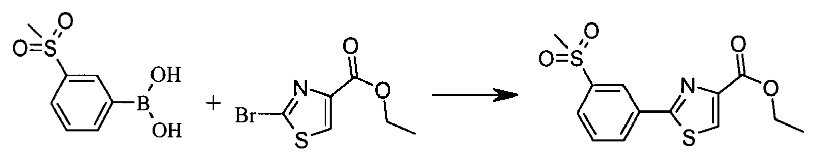

2-Bromthiazol-4-carbonsäureethylester (1 g), 3-(Methylsulfonyl)phenylboronsäure (1.02 g, 1.2 Äquivalente) und Kaliumcarbonat (1.76 g) werden in Dioxan (12.5 ml) vorgelegt. Man gibt (1,3-Diisopropylimidazol-2-yliden)(3-chlorpyridyl)palladium(II) dichlorid (288 mg) zu und die Mischung wird 18 h unter Schutzgas unter Rückfluß gerührt. Danach wird Dioxan am Rotationsverdampfer entfernt und der Rückstand mit Essigester und Wasser aufgenommen. Die organische Phase wird abgetrennt und die wässrige Phase zweimal mit Essigester nachextrahiert. Die vereinigten organischen Phasen werden mit Kochsalzlösung gewaschen und über Natriumsulfat getrocknet und eingeengt. Nach säulenchromatographischer Reinigung an Kieselgel (Essigester/Hexan) erhält man einen weißen Feststoff (629 mg). C₁₃H₁₃NO₄S₂, M = 311.4. LC-MS (ZQ): Rₜ = 1.07, m/z = 312 [M+H]⁺.

### Stufe b: Herstellung 2-(3-Methansulfonyl-phenyl)-thiazol-4-carbonsäure

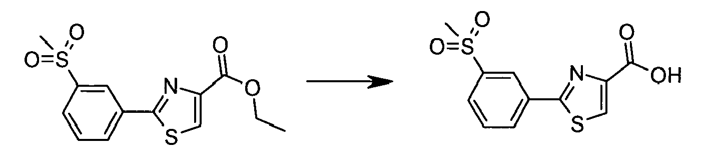

Das Produkt aus Stufe a (629 mg) in THF (12 ml) und Ethanol (12 ml) wird mit Natronlauge (1 M, 20 ml) versetzt und die Mischung wird 3 h bei Raumtemperatur gerührt. Durch Addition von wässriger Salzsäurelösung (1 M) wird die Lösung auf pH = 4 eingestellt. Danach entfernt man die organischen Lösemittel weitestgehend am Rotationsverdampfer und extrahiert die zurückbleibende Lösung mit Essigester, wäscht mit Kochsalzlösung und trocknet mit Natriumsulfat. Durch Einengen im Vakuum erhält man 2-(3-Methansulfonyl-phenyl)-thiazol-4-carbonsäure (241 mg) als Feststoff.
C₁₁H₉NO₄S₂, M = 283.0. LC-MS (ZQ): Rₜ = 1.00, m/z = 284 [M+H]⁺. 1 H-NMR (300 MHz, D6-DMSO): 7.79 (m, 1 H), 8.04 (m, 1 H), 8.27 (m, 1 H), 8.43 (m, 1 H), 8.56 (s, 1 H).

### Stufe c: Herstellung des Endproduktes

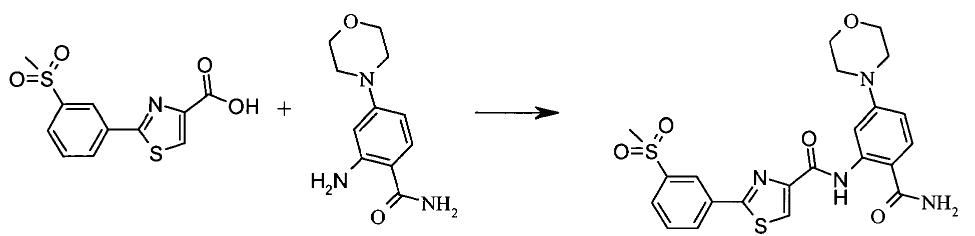

2-(3-Methansulfonyl-phenyl)-thiazol-4-carbonsäure aus Stufe b und 2-Amino-5-(morpholin-4-yl)benzamid (Hour, M.-J., J. Med. Chem. 2000, 43, 4479 - 4487) werden vorgelegt und DMF, Triethylamin und HATU dazu addiert und bei Raumtemperatur gerührt (88.5 h). Nach Zugabe von Wasser wird der ausgefallene Feststoff abfiltriert und mit Essigester und Wasser gewaschen und im Vakuum getrocknet. Man erhält 134 mg eines Feststoffes.

C₂₂H₂₂N₄O₅S₂, M = 486.1. LC-MS (ZQ): Rₜ = 1.01, m/z = 487 [M+H]⁺. 1 H-NMR (300 MHz, D6-DMSO): 3.2 (m, 4H), 3.4 (s), 3.8 (m, 4H), 7.2 (m, 1 H), 7.4 (m, 1 H), 7.7 (s, 1 H), 7.9 (m, 1 H), 8.1 (m, 1 H), 8.3 (br. s, 1 H), 8.4 - 8.6 (m, 3H), 8.7 (m, 1 H), 12.9 (s, 1 H)

### Beispiel 1.4

### 2-(3-Methansulfonyl-phenyl)-thiazol-4-carbonsäure (3-carbamoyl-1-methyl-1H-pyrazol-4-yl)-amid

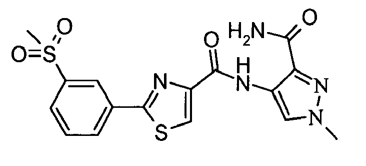

Analog zu Beispiel 1.3, Stufe c, wird 2-(3-Methansulfonyl-phenyl)-thiazol-4-carbonsäure (120 mg) aus Stufe b mit 4-Amino-1-methyl-1 H-pyrazol-3-carbonsäureamid (65 mg) umgesetzt. Nach Reinigen durch präparative HPLC sowie säulenchromatographischer Reinigung an Kieselgel (Laufmittel Cyclohexan/Aceton) erhält man 2-(3-Methansulfonyl-phenyl)-thiazol-4-carbonsäure (3-carbamoyl-1-methyl-1 H-pyrazol-4-yl)-amid (3.4 mg) als weißen Feststoff.

C₁₆H₁₅N₅O₄S₂, M = 405.1; LC-MS (ZQ): Rₜ = 0.92, m/z = 406 [M+H]⁺. 1 H-NMR (300 MHz, D6-DMSO): 3.31 (s, 3H), 3.90 (s, 3H), 7.56 (s, 1 H), 7.72 (s, 1 H), 7.84 (m, 1 H), 8.07 (m, 1 H), 8.32 - 8.38 (m, 2H), 8.43 (m, 1 H), 8.55 (s, 1 H), 11.3 (s, 1 H)

### Beispiel 1.5

### 2-(3-Methansulfonyl-phenyl)-thiazol-4-carbonsäure [2-carbamoyl-4 -(4-methyl-piperazin-1-yl)-phenyl]-amid

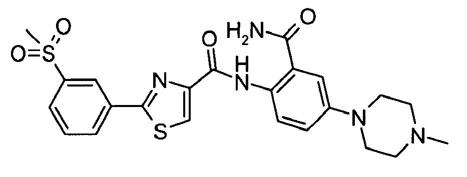

### Stufe a: Herstellung 5-(4-Methyl-piperazin-1-yl)-2-nitro -benzamid (Ausgangsverbindung VII)

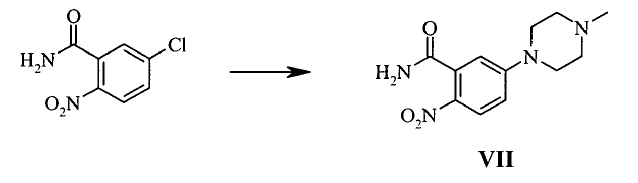

5-Chlornitrobenzamid (7 g) wird in THF vorgelegt und N-Methylpiperazin (4.3 ml, 1.1 Äquivalente) per Spritze zugegeben und unter Rückfluß 22 Stunden erhitzt. Dann werden erneut 1.1 Äq. N-Methylpiperazin zugegeben und 26 h unter Rückfluß erhitzt. Danach gibt man Tetrabutylammoniumfluorid (5 ml, 1 M in THF) und 1.0 Äq. N-Methylpiperazin zu und erhitzt erneut 27.5 h. Man kühlt auf Raumtemperatur ab und saugt den ausgefallenen Feststoff ab. Nach Waschen mit Tetrahydrofuran und Trocknen im Vakuum erhält man Ausgangsverbindung VII als Feststoff (9.4 g, Rohprodukt).
1 H-NMR (400 MHz, D6-DMSO, ausgewählte Signale): δ = 6.82 (d, 1 H), 6.97 (dd, 1 H), 7.48 (br. s, 1 H), 7.83 (br.s, 1 H), 7.90 (d, 1 H).

### Stufe b: Herstellung 2-Amino-5-(4-methyl-piperazin-1-yl) -benzamid (Ausgangsverbindung VIII)

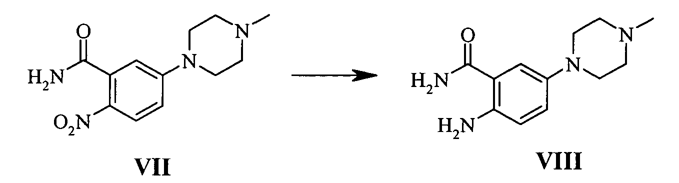

Die Ausgangsverbindung VII (9.4 g) in Ethanol (188 ml) wird mit Palladium auf Kohle (10%ig, wasserfeucht) versetzt. Danach wird bei Raumtemperatur unter einer Wasserstoffatmosphäre 1.5 h gerührt. Der Katalysator wird abfiltriert und Ethanol am Rotationsverdampfer entfernt. Man erhält VIII als Feststoff (Rohprodukt).

1 H-NMR (400 MHz, D6-DMSO): δ = 2.78 (s, 3H), 2.9 - 3.7 (m, zum Teil verdeckt durch Wassersignal), 6.0 - 6.5 (br s), 6.65 (d, 1 H), 6.95 (dd, 1 H), 7.07 (br. s, 1 H), 7.11 (d, 1 H), 7.81 (br. s, 1 H)

### Stufe c: Herstellung des Endproduktes

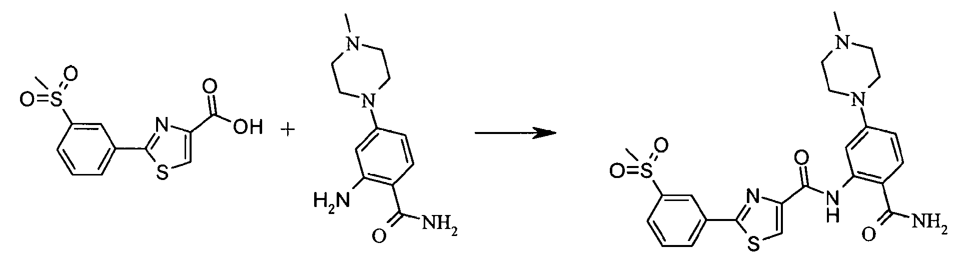

2-(3-Methansulfonyl-phenyl)-thiazol-4-carbonsäure (siehe Beispiel 1.3, Stufe b) (300 mg) und Ausgangsverbindung VIII (273 mg) werden in DMF (7.5 ml) vorgelegt und man addiert HATU (523 mg, 1.3 eq.) und lässt bei Raumtemperatur rühren über Nacht. Man gießt auf Wasser und saugt den ausgefallenen Feststoff ab und wäscht mit Ether nach. Nach Trocknen im Vakuum erhält man Beispiel 1.5 (188 mg) als Feststoff

C₂₃H₂₅N₅O₄S₂, M = 499.6. 1 H-NMR (300 MHz, D6-DMSO): δ = 2.20 (s, 3H), 2.43 (m, zum Teil verdeckt durch Lösemittelsignal), 3.15 (m, 4H), 3.33 (s, 3H), 7.14 (m, 1 H), 7.30 (m, 1 H), 7.67 (s, 1 H), 7.82 (t, 1 H), 8.06 (d, 1 H), 8.26 (s, 1 H), 8.39 - 8.55 (m, 4H), 12.8 (s, 1 H).

### Verfahrensvariante 2

### Beispiel 2.1

### N-[5-(Aminocarbonyl)-1-methyl-1H-pyrazol-4-yl]-2-(2,3-dihydro-5-benzofuranyl)-4-thiazolcarboxamid

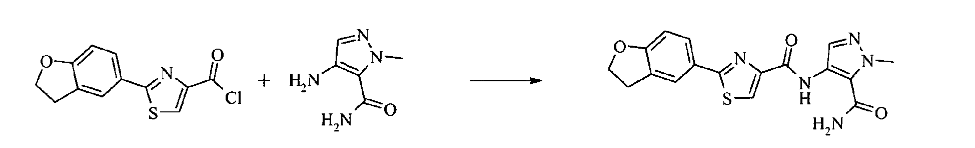

Man legt 4-Amino-2-methyl-2H-pyrazol-3-carbonsäureamid (35 mg) in Dichlormethan (1 ml) und Dimethylacetamid (0.5 ml) vor und addiert Triethylamin (50 Microliter) und 4-Dimethylaminopyridin (DMAP) (Spatelspitze) sowie 2-(2,3-Dihydro-1-benzofuran-5-yl)-1,3-thiazol-4-carbonylchlorid CAS 306936-10-7 (66 mg) und lässt bei Raumtemperatur 18.5 h rühren. Danach entfernt man die Lösemittel im Vakuum und erhält nach HPLC Beispiel 2.1 als weißen Feststoff (35 mg).

C₁₇H₁₅N₅O₃S, M = 369.4, LC-MS (ZQ): Rₜ = 1.01, m/z = 370 [M+H]⁺.

### Beispiel 2.2

### N-[2-(Aminocarbonyl)-4-methylphenyl]-2-(2,3-dihydro-5-benzofuranyl)-4-thiazolcarboxamid

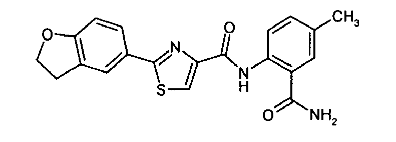

Analog zur Synthese von Beispiel 2.1 erhält man N-[2-(Aminocarbonyl)-4-methylphenyl]-2-(2,3-dihydro-5-benzofuranyl)-4-thiazolcarboxamid als Feststoff durch die Reaktion von 2-(2,3-Dihydro-1-benzofuran-5-yl)-1,3-thiazol-4-carbonylchlorid und 2-Amino-5-methylbenzamid.

C₂₀H₁₇N₃O₃S, M = 379.4, LC-MS (ZQ): Rₜ = 1.22, m/z = 380 [M+H]⁺.

### Beispiel 2.3

### N-[2-(Aminocarbonyl)-4-chlorphenyl]-2-(2,3-dihydro-5-benzofuranyl)-4-thiazolcarboxamid

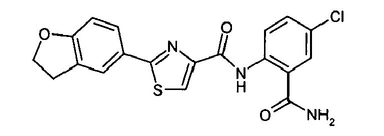

Analog zur Synthese von Beispiel 2.1 erhält man N-[2-(Aminocarbonyl)-4-chlorphenyl]-2-(2,3-dihydro-5-benzofuranyl)-4-thiazolcarboxamid als Feststoff durch die Reaktion von
2-(2,3-Dihydro-1-benzofuran-5-yl)-1,3-thiazol-4-carbonylchlorid und 2-Amino-5-chlorbenzamid.

C₁₉H₁₄ClN₃O₃S, M = 399.9. LC-MS (ZQ): Rₜ = 1.29 min, m/z = 400 [M+H]⁺.

### Beispiel 2.4

### N-[2-(Aminocarbonyl)phenyl]-2-(2,3-dihydro-5-benzofuranyl)-4-thiazolcarboxamid

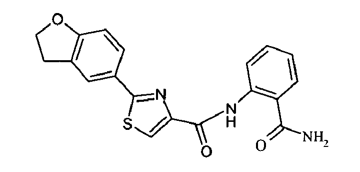

Analog zur Synthese von Beispiel 2.1 erhält man N-[2-(Aminocarbonyl)phenyl]-2-(2,3-dihydro-5-benzofuranyl)-4-thiazolcarboxamid als Feststoff durch die Reaktion von 2-(2,3-Dihydro-1-benzofuran-5-yl)-1,3-thiazol-4-carbonylchlorid und 2-Aminobenzamid.

C₁₉H₁₅N₃O₃S, M = 365.4. 1 H-NMR (300 MHz, D6-DMSO): δ = 3.29 (t, 2H), 4.65 (t, 2H), 6.93 (m, 1 H), 7.18 (m, 1 H), 7.56 (m, 1 H), 7.80 - 7.88 (m, 2H), 7.91 (m, 1 H), 7.95 (s, 1H), 8.28 (s, 1H), 8.37 (s, 1H), 8.83 (m, 1 H), 13.1 (s, 1 H).

### Beispiel 2.5

### 2-{[2-(2,3-Dihydro-benzofuran-5-yl)-thiazol-4-carbonyl]-amino}-6-methyl-4,5,6,7-tetrahydro-thieno[2,3-c]pyridin-3-carbonsäureamid

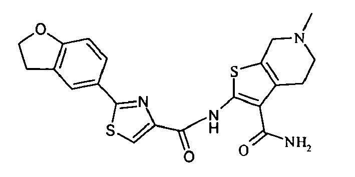

Analog zur Synthese von Beispiel 2.1 erhält man 2-{[2-(2,3-Dihydro-benzofuran-5-yl)-thiazol-4-carbonyl]-amino}-6-methyl-4,5,6,7-tetrahydro-thieno[2,3-c]pyridin-3-carbonsäureamid als Feststoff durch die Reaktion von 2-(2,3-Dihydro-1-benzofuran-5-yl)-1,3-thiazol-4-carbonylchlorid und 2-Amino-6-methyl-4,5,6,7-tetrahydrothieno[2,3-c]pyridin-3-carbonsäureamid.

C₂₁H₂₀N₄O₃S₂, M = 440.5. LC-MS: Rₜ = 2.98, m/z = 441.5 [M+H]⁺. Aufnahme durch ein Autopurifier HPLC-System der Firma *Waters* (1525µ Binary HPLC Pump, *(Waters),* Detector: MS: Micromass ZQ, Hersteller: *(Waters),* UV- Lampe: MUX UV 2488 Detector, Hersteller: (*Waters*), Wellenlänge: 210-350 nm, Säule: XBridge *(Waters),* 4,6x50 mm, C18 3,5µm, Gradient: Acetonitril / Wasser (enthält 0.1 % TFA), von: 1 % Acetonitril bis 99%, Runtime: 8 min., Flussrate:2 ml/min.

### Verfahrensvariante 3

### Beispiel 3.1 N-[2-(Aminocarbonyl)phenyl]-2-(3,4-methylendioxyphenyl)-4-thiazolcarboxamid

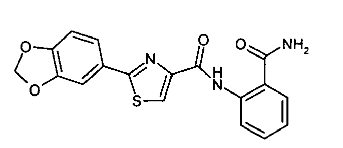

### a) Herstellung des Intermediates V.1 N-[2-(Aminocarbonyl)phenyl]-2-brom-4-thiazolcarboxamid

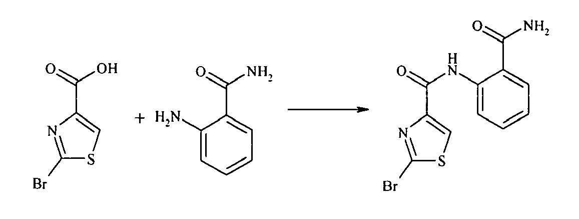

2-Brom-4-thiazolcarbonsäure (1 g), HATU (2.01 g) und 2-Aminobenzamid (0.65 g) in N,N-Dimethylformamid (DMF) (20 ml) werden vorgelegt. Man kühlt mit einem Eisbad ab und gibt N,N-Diisopropylethylamin (0.90 ml) zu. Man lässt 6 Tage bei Raumtemperatur rühren, gießt das Reaktionsgemisch auf Eiswasser, lässt unter Rühren auftauen und saugt den ausgefallenen Feststoff ab, wäscht zweimal mit Wasser, zweimal mit Diethylether und trocknet im Vakuum. Man erhält das Intermediat V.1 als Feststoff (1.4 g).

C₁₁H₈BrN₃O₂S, M = 326.2. 1 H-NMR (300 MHz, D6-DMSO): δ = 7.20 (m, 1 H), 7.56 (m, 1 H), 7.79 (s, 1 H), 7.84 (m, 1 H), 8.30 (s, 1 H), 8.47 (m, 1 H), 8.67 (m, 1 H), 12.9 (s, 1 H).

### b) Herstellung des Endproduktes

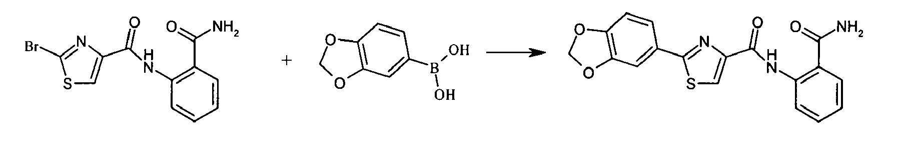

Das Intermediat V.1 (65 mg) und 3,4-Methylendioxyphenylboronsäure (46 mg, 1.4 Äquivalente) werden in Toluol (1.5 ml), Ethanol (1.5 ml) und wässriger Natriumcarbonatlösung (180 Microliter, 2M) vorgelegt. Man gibt Pd(PPh₃)₄ (23 mg) zu und erhitzt in der Microwelle (Gerät CEM Explorer, 120°C, 20 min, 300 Watt). Danach gibt man Wasser und Essigester zu, rührt um und trennt die organische Phase ab und engt diese in einer Zentrifuge ein. Der resultierende Feststoff wird durch präparative HPLC gereinigt.

C₁₈H₁₃N₃O₄S, M = 367.4, LC-MS (ZQ): Rₜ = 1.15, m/z = 368 [M+H]⁺.

### Beispiel 3.2 N-[2-(Aminocarbonyl)phenyl]-2-(3-methylsulfonylphenyl)-4-thiazolcarboxamid

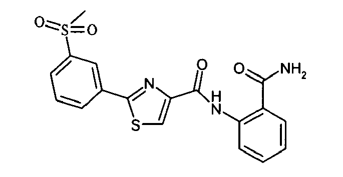

Man legt (analog zu C. J. O'Brien et. al. Chem. Eur. J. 2006, 12, 4743 - 4748) Intermediat V.1 (65 mg) und 3-Methylsulfonylphenylboronsäure (80 mg) in Dioxan (2 ml) vor und addiert Kaliumcarbonat (83 mg) und 1,3-Bis(2,6-diisopropylphenyl)imidazol-2-yliden(3-chlorpyridyl)palladium(II)dichlorid (10 mg). Nach Erhitzen in der Mikrowelle (120°C, 30 min, 300W) und Aufarbeitung wie bei der Beispiel 3.1 wird N-[2-(Aminocarbonyl)phenyl]-2-(3-methylsulfonylphenyl)-4-thiazolcarboxamid als weißer Feststoff erhalten.

C₁₈H₁₅N₃O₄S₂, M = 401.5. LC-MS (ZQ): Rₜ = 1.01, m/z = 402 [M+H]⁺.

### Beispiel 3.3 N-[2-(Aminocarbonyl)phenyl]-2-(3-cyanophenyl)-4-thiazolcarboxamid

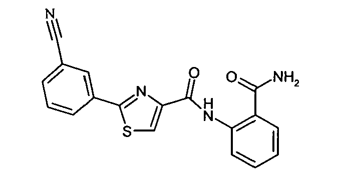

Analog zu Beispiel 3.2 erhält man N-[2-(Aminocarbonyl)phenyl]-2-(3-cyanophenyl)-4-thiazolcarboxamid aus Intermediat V.1 und 3-Cyanophenylboronsäure.

C₁₈H₁₂N₄O₂S, M = 348.4. LC-MS (ZQ): Rₜ = 1.11, m/z = 349 [M+H]⁺.

### Beispiel 3.4 N-[2-(Aminocarbonyl)phenyl]-2-(3-fluoro-4-methoxyphenyl)-4-thiazolcarboxamid

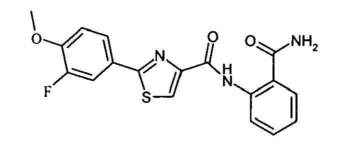

Analog Beispiel 3.2 erhält man N-[2-(Aminocarbonyl)phenyl]-2-(3-fluoro-4-methoxyphenyl)-4-thiazolcarboxamid aus Intermediat V.1 und 3-Fluoro-4-methoxyphenylboronsäure.

C₁₈H₁₄FN₃O₃S, M = 371.4. 1 H-NMR (400 MHz, D6-DMSO): 3.91 (s, 3H), 7.15 (m, 1 H), 7.29 (m, 1 H), 7.53 (m, 1 H), 7.77 -7.88 (3H), 7.94 (m, 1 H), 8.26 (s, 1 H), 8.41 (s, 1 H), 8.70 (m, 1 H), 13.2 (s, 1 H).

### Beispiel 3.5

### N-[2-(Aminocarbonyl)-5-methylphenyl]-2-(4-methyl-3,4-dihydro-2H-benzo[1,4]oxazin-7-yl)-thiazol-4-carbonsäureamid

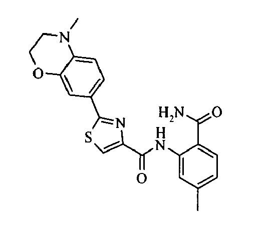

### a) Herstellung des Intermediates V.2 N-[2-(Aminocarbonyl)-5-methylphenyl]-2-brom-4-thiazolcarboxamid

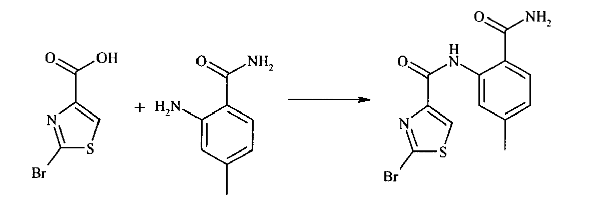

Analog zur Synthese von Intermediat V.1 wird 2-Brom-4-thiazolcarbonsäure und 2-Amino-4-methylbenzamid zu N-[2-(Aminocarbonyl)-5-methylphenyl]-2-brom-4-thiazolcarboxamid umgesetzt.

C₁₂H₁₀BrN₃O₂S, M = 339.0, LC-MS (ZQ): Rₜ = 1.04, m/z = 340 [M+H]⁺. 1 H-NMR (300 MHz, D6-DMSO): δ = 2.32 (s, 3H), 6.97 (m, 1 H) 7.65 (br. s., 1 H), 7.70 (d, 1 H), 8.18 (br. s., 1 H), 8.40 (s, 1 H), 8.49 (m, 1 H), 13.0 (s, 1 H).

### b) Herstellung des Endproduktes

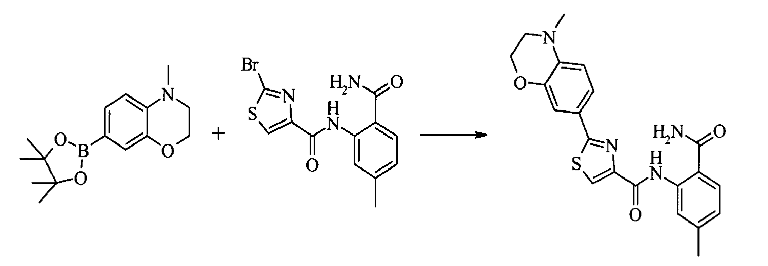

Man legt Intermediat V.2 (150 mg, 0.44 mmol) und 4-Methyl-7-(4,4,5,5-tetramethyl-1,3,2-dioxaborolan-2-yl)-3,4-dihydro-2H-1,4-benzoxazin (147 mg, 0.53 mmol) in Ethylenglykoldimethylether (3 ml) und wässriger Natriumhydrogencarbonatlösung (2 ml, 2M) vor, gibt Pd(PPh₃)₄ (51 mg, 0.044 mmol) zu und erhitzt in der Mikrowelle 45 min bei 110°C (300W) (Gerät CEM Explorer). Nach weiterer Zugabe von Pd(PPh₃)₄ (51 mg, 0.044 mmol) erhitzt man erneut 30 min bei 110°C. Das Lösemittel wird abdestilliert und der Rückstand in Essigester und Wasser aufgenommen, die organische Phase abgetrennt, die wässrige Phase mit Essigester extrahiert, die vereinigten organischen Phasen mit gesättigter Kochsalzlösung gewaschen und über Natriumsulfat getrocknet. Nach Einengen am Rotationsverdampfer wird der Rückstand per HPLC gereinigt.
C₂₁H₂₀N₄O₃S, M = 408.1, LC-MS (ZQ): Rₜ = 1.39, m/z = 409 [M+H]⁺. 1 H-NMR (400 MHz, D6-DMSO): 2.33 (s, 3H), 2.91 (s, 3H), 3.33 (m, überlagert durch Wassersignal), 4.23 (m, 2H), 6.72 (m, 1 H), 6.95 (m, 1 H), 7.39 (m, 1 H), 7.46 (m, 1 H), 7.67 - 7.75 (m, 2H), 8.15 (br. s, 1 H), 8.22 (s, 1 H), 8.57 (s, 1 H), 13. 1 (s, 1 H).

## Patentansprüche

1. Verbindungen der allgemeinen Formel (I) mit den Bausteinen A, B, C und D , 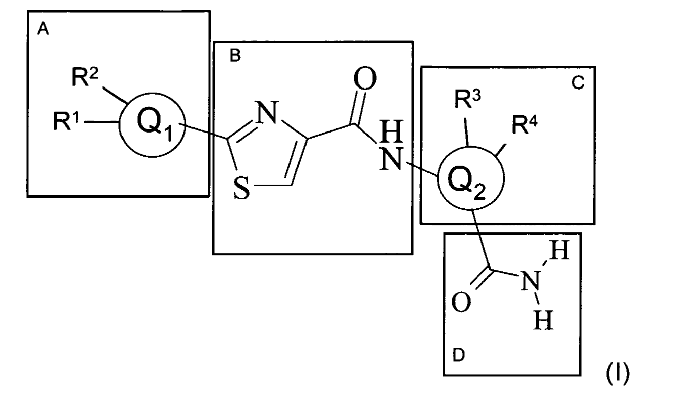 in der
die Bausteine B und D in ortho-Stellung zueinander stehen und
Q₁ für einen Phenylring oder hydrierten bicyclischen Heteroarylring steht,
R¹ und R² unabhängig voneinander stehen für
(v) Wasserstoff, Hydroxy, Nitro, Halogen, Cyano, -CF₃, -NR⁵R⁶ oder
(vi) -C(O)-OR¹⁰, -C(O)-R⁷, -C(O)-C₁-C₃-Fluoralkyl, -C(O)-NR⁵R⁶, -NH-C(O)-R⁷ oder
(vii) einen C₁-C₆-Alkyl-, C₂-C₆ -Alkenyl-, C₂-C₆-Alkinyl-, C₁-C₆-Alkoxy-, C₁-C₆- Fluoralkyl- oder einen C₁-C₆- Fluoralkoxyrest, jeweils gegebenenfalls mit -C₁-C₃-Alkoxy, Hydroxy, -C(O)-OR¹⁰ oder -NR⁸R⁹ ein- oder mehrfach, gleich oder verschieden substituiert, oder
(viii) -O-SO₂-NR⁵R⁶, -SO₂-R⁷, -SO₂-NR⁵R⁶, Q₂ für einen Aryl-, Heteroaryl- oder einen hydrierten bizyklischen Heteroarylring steht;
R³ und R⁴ unabhängig voneinander stehen für
(iii) Wasserstoff, Halogen oder -NR¹¹R¹², oder
(iv) einen C₁-C₆-Alkyl- oder C₁-C₆-Alkoxyrest, die jeweils gegebenenfalls ein- oder mehrfach, gleich oder verschieden mit Hydroxy, C₁-C₃-Alkoxy, -NR⁸R⁹ oder Heterocyclyl substituiert sind, wobei der Heterocylylring ein- oder mehrfach, gleich oder verschieden substituiert sein kann mit C₁-C₃-Alkyl oder -CO-R⁷,
R⁵ und R⁶ unabhängig voneinander stehen für Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder-CO-R⁷, oder
R⁵ und R⁶ bilden alternativ gemeinsam mit dem Stickstoffatom einen 5- bis 7-gliedrigen Ring, der gegebenenfalls zusätzlich zum Stickstoffatom ein weiteres Heteroatom enthält und der gegebenenfalls mit C₁-C₆-Alkyl und/oder mit -C(O)-R⁷ ein- oder mehrfach, gleich oder verschieden substituiert ist, und
R⁷ für einen C₁-C₆ Alkylrest steht, und
R⁸, R⁹, R¹⁰ unabhängig voneinander stehen für Wasserstoff oder einen C₁-C₆ Alkylrest, und
R¹¹ und R¹² unabhängig voneinander stehen für Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder-CO-R⁷, oder
R¹¹ und R¹² bilden alternativ gemeinsam mit dem Stickstoffatom einen 5- bis 7-gliedrigen Ring, der gegebenenfalls zusätzlich zum Stickstoffatom ein weiteres Heteroatom enthält und der gegebenenfalls mit C₁-C₆-Alkyl und/oder mit -C(O)-R⁷ ein- oder mehrfach, gleich oder verschieden substituiert ist,
sowie deren Salze, Enantiomere und Diastereomere.

2. Verbindungen der allgemeinen Formel (I) mit den Bausteinen A, B, C und D, in der
die Bausteine B und D in ortho-Stellung zueinander stehen und
Q₁ für einen Phenyl-, Benzodioxolyl-, 3,4-Methylendioxyphenyl-, 2,3-Dihydrobenzofuranyl-, 3,4-Dihydro-2H-benzo[1,4]oxazin-7-yl-, Indanonyl oder einen 2,3-Dihydroindolylring steht,
R¹ und R² unabhängig voneinander stehen für
(i) Wasserstoff, Hydroxy, Halogen, Cyano, -CF₃, -NR⁵R⁶ oder
(ii) einen C₁-C₆-Alkyl-, C₁-C₆-Alkoxy-, C₁-C₆-Fluoralkyl- oder einen C₁-C₆-Fluoralkoxyrest oder
(iii) SO₂-R⁷,
Q₂ Phenyl-, Thienyl-, Thiazolyl-, Furanyl-, Pyrrolyl-, Oxazolyl-, Imidazolyl-, Pyrazolyl-, Isoxazolyl, Isothiazolyl, 1,2,3-Triazolyl, Pyridinyl-, Pyridazinyl-, Pyrimidinyl-, Pyrazinyl-, 1,2,3,4-Tetrahydro-isochinolinyl-, 1,2,3,4-Tetrahydro-chinolinyl-, 2,3-Dihydro-1H-indolyl-, 2,3-Dihydro-1H-isoindolyl-, 4,5,6,7-Tetrahydro-thieno[2,3-c]pyridinyl-, 4,5,6,7-Tetrahydro-thieno[3,2-c]pyridinyl-, 2,3-Dihydro-benzofuranyl-, Benzo[1,3]dioxolyl-, 2,3-Dihydro-benzo[1,4]dioxinyl-, 1,2,3,4-Tetrahydro-chinoxalinyl- oder einen 3,4-Dihydro-2H-benzo[1,4]oxazinylring steht,
R³ und R⁴ unabhängig voneinander stehen für
(i) Wasserstoff, Halogen, -NR¹¹R¹² oder
(ii) einen C₁-C₆-Alkyl- oder C₁-C₆-Alkoxyrest, jeweils gegebenenfalls mit Morpholin oder -NR⁸R⁹ substituiert,
wobei
R⁵ und R⁶ unabhängig voneinander stehen für Wasserstoff oder einen C₁-C₆ Alkylrest, der gegebenenfalls ein- oder mehrfach, gleich oder verschieden mit Hydroxy, -NR⁸R⁹, oder C₁-C₃-Alkoxy substituiert sein kann, und
R⁷ für einen C₁-C₄ Alkylrest steht, und
R⁸ und R⁹ unabhängig voneinander stehen für Wasserstoff oder einen C₁-C₄ Alkylrest,
R¹¹ und R¹² unabhängig voneinander stehen für Wasserstoff oder einen C₁-C₆ Alkylrest, der gegebenenfalls ein- oder mehrfach, gleich oder verschieden mit Morpholin oder durch -N(CH₃)₂, -NH-CH₃ oder -NH-C₂H₅ substituiert sein kann, oder
R¹¹ und R¹² bilden alternativ gemeinsam mit dem Stickstoffatom einen 5- bis 7-gliedrigen Ring, der gegebenenfalls zusätzlich zum Stickstoffatom ein weiteres Heteroatom enthält und der gegebenenfalls mit C₁-C₄-Alkyl und/oder mit -C(O)-R⁷ ein- oder mehrfach, gleich oder verschieden substituiert ist,
sowie deren Salze, Enantiomere und Diastereomere.

3. Verbindungen der allgemeinen Formel (I) mit den Bausteinen A, B, C und D gemäß Anspruch 1 oder 2, in der
Q₁ für einen für Phenyl-, 3,4-Methylendioxyphenyl-, 2,3-Dihydrobenzofuranyl- oder einen 3,4-Dihydro-2H-benzo[1,4]oxazin-7-ylring steht,
sowie deren Salze, Enantiomere und Diastereomere.

4. Verbindungen der allgemeinen Formel (I) mit den Bausteinen A, B, C und D gemäß einem der Ansprüche 1 bis 3, in der
R¹ und R² unabhängig voneinander stehen für
(i) Wasserstoff, Halogen, oder
(ii) einen C₁-C₆-Alkoxy-, C₁-C₆-Alkylrest, oder
(iii) -SO₂-R⁷
wobei R⁷ für einen C₁-C₃-Alkylrest steht,
sowie deren Salze, Enantiomere und Diastereomere.

5. Verbindungen der allgemeinen Formel (I) mit den Bausteinen A, B, C und D gemäß einem der Ansprüche 1 bis 4, in der
Q₂ für einen Phenyl-, Pyrazolyl- oder einen 4,5,6,7-Tetrahydro-thieno[2,3-c]pyridinylring steht,
sowie deren Salze, Enantiomere und Diastereomere.

6. Verbindungen der allgemeinen Formel (I) mit den Bausteinen A, B, C und D gemäß einem der Ansprüche 1 bis 5, in der
R³ und R⁴ unabhängig voneinander stehen für Wasserstoff, Halogen, C₁-C₆-Alkyl oder -NR¹¹R¹², wobei
R¹¹ und R¹² gemeinsam mit dem Stickstoffatom einen 6-gliedrigen Ring bilden, der gegebenenfalls zusätzlich zum Stickstoffatom ein weiteres Heteroatom enthält und der mit C₁-C₄-Alkyl einoder mehrfach, gleich oder verschieden substituiert sein kann,
sowie deren Salze, Enantiomere und Diastereomere.

7. Verbindungen der allgemeinen Formel (I) mit den Bausteinen A, B, C und D in der die Bausteine B und D in ortho-Stellung zueinander stehen und
Q₁ für einen für Phenyl-, 3,4-Methylendioxyphenyl-, 2,3-Dihydrobenzofuranyl- oder einen 3,4-Dihydro-2H-benzo[1,4]oxazin-7-ylring steht,
R¹ und R² unabhängig voneinander stehen für
(i) Wasserstoff, Halogen, oder
(ii) einen C₁-C₆-Alkoxy-, C₁-C₆-Alkylrest, oder
(iii) -SO₂-R⁷
Q₂ für einen für einen Phenyl-, Pyrazolyl- oder einen 4,5,6,7- Tetrahydro-thieno[2,3-c]pyridinylring steht,
R³ und R⁴ unabhängig voneinander stehen für Wasserstoff, Halogen, C₁-C₆-Alkyl oder -NR¹¹R¹²,
wobei
R⁷ für einen C₁-C₃ -Alkylrest steht, und
R¹¹ und R¹² gemeinsam mit dem Stickstoffatom einen 6-gliedrigen Ring bilden, der gegebenenfalls zusätzlich zum Stickstoffatom ein weiteres Heteroatom enthält und der mit C₁-C₄-Alkyl ein- oder mehrfach, gleich oder verschieden substituiert sein kann,
sowie deren Salze, Enantiomere und Diastereomere.

8. Verbindungen nach einem der Ansprüche 1 bis 7, nämlich
N-[2-(Aminocarbonyl)-4-(4-methyl-piperazin-1-yl)phenyl]-2-(3-chlorphenyl)-4-thiazolcarboxamid
N-[2-Aminocarbonyl-4-(4-methyl-piperazin-1-yl)-phenyl]-2-phenyl-4-thiazolcarboxamid
2-(3-Methansulfonyl-phenyl)-thiazol-4-carbonsäure (2-carbamoyl-4 -(morpholin-4-yl)-phenyl)-amid
2-(3-Methansulfonyl-phenyl)-thiazol-4-carbonsäure (3-carbamoyl-1-methyl-1 H-pyrazol-4-yl)-amid
2-(3-Methansulfonyl-phenyl)-thiazol-4-carbonsäure [2-carbamoyl-4 -(4-methyl-piperazin-1-yl)-phenyl]-amid
N-[5-(Aminocarbonyl)-1-methyl-1 H-pyrazol-4-yl]-2-(2,3-dihydro-5-benzofuranyl)-4-thiazolcarboxamid
N-[2-(Aminocarbonyl)-4-methylphenyl]-2-(2,3-dihydro-5-benzofuranyl)-4-thiazolcarboxamid
N-[2-(Aminocarbonyl)-4-chlorphenyl]-2-(2,3-dihydro-5-benzofuranyl)-4-thiazolcarboxamid
N-[2-(Aminocarbonyl)phenyl]-2-(2,3-dihydro-5-benzofuranyl)-4-thiazolcarboxamid
2-{[2-(2,3-Dihydro-benzofuran-5-yl)-thiazol-4-carbonyl]-amino}-6-methyl-4,5,6,7-tetrahydro-thieno[2,3-c]pyridin-3-carbonsäureamid
N-[2-(Aminocarbonyl)phenyl]-2-(3,4-methylendioxyphenyl)-4-thiazolcarboxamid
N-[2-(Aminocarbonyl)phenyl]-2-(3-methylsulfonylphenyl)-4-thiazolcarboxamid
N-[2-(Aminocarbonyl)phenyl]-2-(3-cyanophenyl)-4-thiazolcarboxamid
N-[2-(Aminocarbonyl)phenyl]-2-(3-fluoro-4-methoxyphenyl)-4-thiazolcarboxamid
N-[2-(Aminocarbonyl)-5-methylphenyl]-2-(4-methyl-3,4-dihydro-2H-benzo[1,4]oxazin-7-yl)-thiazol-4-carbonsäureamid

9. Verfahren zur Herstellung von Verbindungen gemäß einem der Ansprüche 1 bis 8 **gekennzeichnet durch** die Umsetzung von Intermediaten der Formel (III) mit Intermediaten der Formel (II) mit Hilfe eines
Amidkupplungsreagenzes. 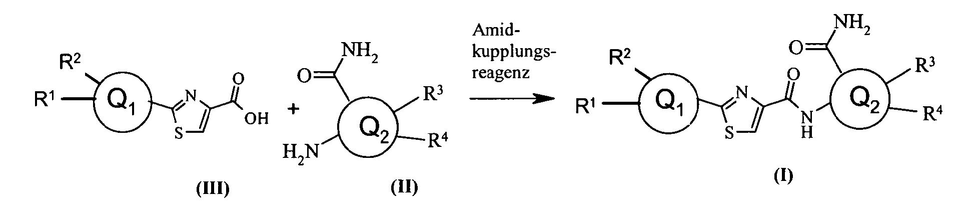 wobei
Q₁, Q₂, R¹, R², R³ und R⁴ die in der allgemeinen Formel (I) gemäß der Ansprüche 1 bis 7 angegebenen Bedeutungen besitzen.

10. Verfahren zur Herstellung von Verbindungen gemäß einem der Ansprüche 1 bis 8 **gekennzeichnet durch** die Umsetzung von Intermediaten der Formel (IV) mit Intermediaten der Formel (II) in Gegenwart einer Base. 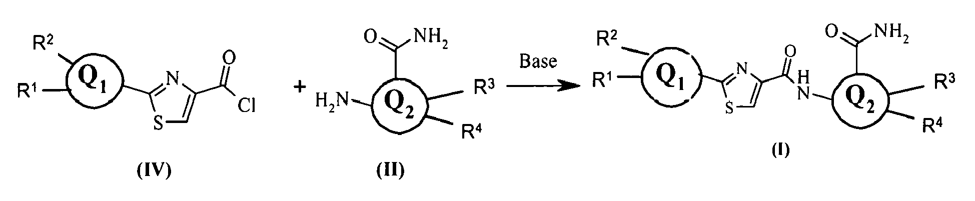 wobei
Q₁, Q₂, R¹, R², R³ und R⁴ die in der allgemeinen Formel (I) gemäß der Ansprüche 1 bis 7 angegebenen Bedeutungen besitzen.

11. Verfahren zur Herstellung von Verbindungen gemäß einem der Ansprüche 1 bis 8 mit den Teilschritten
a) Umsetzung von 2-Bromthiazol-4-carbonsäure mit Intermediaten der Formel (II) durch ein Amidkupplungsreagenz zu Intermediaten der Formel (V) 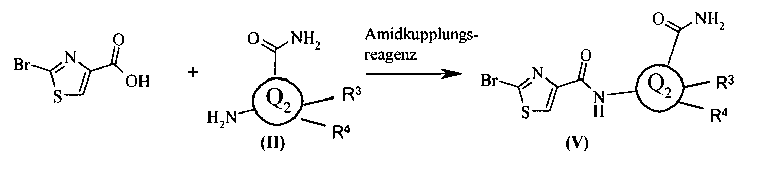
b) Umsetzung der Intermediate der Formel (V) mit Boronsäuren oder Boronsäurepinakolestern im Rahmen einer *Suzuki-Miyaura-Reaktion* zu Verbindungen der Formel (I) 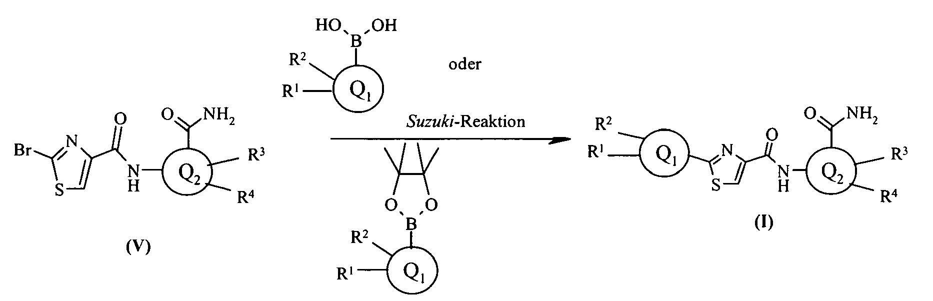
wobei
Q₁, Q₂, R¹, R², R³ und R⁴ die in der allgemeinen Formel (I) gemäß der Ansprüche 1 bis 7 angegebenen Bedeutungen besitzen.
